# EUROPEAN PATENT APPLICATION

(11) **EP 4 847 065 A2**
(43) Date of publication of application: **02.12.2026**
(21) Application number: 26213079.2
(22) Date of filing: 27.07.2021
(51) Int. Cl.: A61M 5/32

(54) **DOWNSTREAM PLASMA TREATED SILICONIZED PLASTIC SYRINGE BARREL AND RELATED SYRINGES AND METHODS**

(30) Priority: 27.07.2020 US 202063057284 P
(62) Document number(s) of the earlier application(s) in accordance with Art. 76 EPC: 21849297.3 / 4 188 670
(71) Applicant: TriboFilm Research, Inc., Raleigh, NC 27606 (US)
(72) Inventor: THORNTON, Jackson, D., Raleigh, NC, 27609 (US); SAKHRANI, Vinay, Raleigh, NC, 27612 (US)
(74) Representative: Piotrowicz, Pawel Jan Andrzej

(57) **Abstract**

A syringe comprises a downstream plasma-treated plastic syringe barrel having an interior surface coated with a downstream plasma-treated polysiloxane-based lubricant coating, a plunger rod, a plunger stopper and a needle. When the syringe barrel is empty, its coated interior surface has a surface density of no more than 50 particles per cm² for particles greater than 8 µm in diameter. The syringe contains a solution having no more than 50 particles per mL with a diameter of at least 10 µm, or no more than 5 particles per mL with a diameter of at least 25 µm.

## Description

### CROSS REFERENCE TO RELATED APPLICATIONS

This application claims priority to U.S. Provisional 63/057,284 filed on July 27, 2020, the contents of which are hereby incorporated by reference in their entirety.

### STATEMENT REGARDING FEDERALLY SPONSORED RESEARCH OR DEVELOPMENT

This invention was made with government support under Grant No. R44EY024461, awarded by the National Eye Institute, an institute of the National Institutes of Health. The United States has certain rights in the invention.

### FIELD

The invention relates to plastic syringes used for delivery of medicaments.

### BACKGROUND

As the biopharmaceutical market continues to grow, the need for parenteral dosage forms increases. Pre-filled syringes and drug products supplied in glass vials for administration with general use syringes are the most common packaging configurations. Syringes are typically made either of glass or plastic.

Current trends with respect to pre-filled syringes lean towards use of glass pre-filled syringes with baked on silicone (e.g. Gerrescheimer Gx^{®} Baked-on RTF^{®} glass syringes), plastic pre-filled syringes with chemically cross-linked silicone (Schott TopPac^{®}), and silicone-free plastic syringe systems (e.g. Terumo PLAJEX^{™}, West Daikyo CZ^{®} and BD Sterifill^{™}). However, these unassembled, nested prefillable syringes and the associated manufacturing equipment - even apart from the cost of the biologic drug itself - can be cost prohibitive.

User-fillable plastic syringes and compounded drug products in assembled general use plastic syringes offer the advantage of significantly lower costs (sometimes as much as a 95% cost reduction) as compared to pre-filled syringe formats. However, general use plastic syringes are associated with subvisible particulates derived from silicone oil used for lubrication to enable the plunger stopper to glide inside the syringe barrel, and protein aggregation induced by subvisible silicone oil particles after filling with a drug. Siliconized plastic pre-filled syringes as well are associated with silicone oil subvisible particles, such that glass pre-filled syringes continue to make up a significant portion of the pre-filled syringes market (roughly 70%) and product development of plastic pre-filled syringes is focused on silicone-free plastic syringes.

There remains a need for simple and safe solutions that can take advantage of the stability and safety afforded by lyophilization of biological drugs in vials and the extremely low cost of general use plastic syringes for bedside medication administration or compounded drugs, while providing a more stable silicone oil layer that minimizes subvisible particle levels.

Plastic syringes are also used for intravitreal injections into the eye for treatment of Macular Degeneration and Diabetic Retinopathy. These expensive biologic drugs are supplied in vials and need to be filled in general use syringes before administration. The silicone oil used in these general use syringes introduce particulate contaminants in the drug solution that get injected into the eyes of patients. Several complications ranging from increased intraocular pressure to complaints about visual floaters have been reported due to these silicone oil particles. FDA requires ophthalmic solutions to meet their USP789 guidance for particulate contamination. USP789 requires there to be no more than 50 particles per ml ≥ 10 microns and 5 particles per ml ≥ 25 microns. While the biologic solutions in the vials have to meet the USP789 requirements, the syringes used to administer these drugs are not regulated and introduce particulate contaminants that cause patient complications. There remains a need for low particulate plastic syringes for injections of ophthalmic solutions that are supplied in vials.

### SUMMARY OF THE INVENTION

The presently disclosed subject matter describes a plastic syringe barrel comprising a plastic syringe barrel treated with plasma and coated with a polysiloxane-based lubricant coating treated with plasma, wherein each plasma treatment consisted essentially of uncharged energized gaseous species; and wherein the interior surface of the plasma-treated plastic syringe barrel with the plasma-treated polysiloxane-based lubricant coating has at least a 75%, 80%, 85%, 90%, or 95% reduction in the number of particles greater than 8 microns in diameter as compared to an interior surface of a plastic syringe barrel with a polysiloxane-based lubricant coating. The presently disclosed subject matter describes a plastic syringe barrel comprising a plastic syringe barrel treated with plasma and coated with a polysiloxane-based lubricant coating treated with plasma, wherein each plasma treatment comprises a downstream plasma generated at atmospheric pressure; and wherein the interior surface of the plasma-treated plastic syringe barrel with the plasma-treated polysiloxane-based lubricant coating has at least a 75%, 80%, 85%, 90%, or 95% reduction in the number of particles greater than 8 microns in diameter as compared to an interior surface of a plastic syringe barrel with a polysiloxane-based lubricant coating.

The presently disclosed subject matter describes a plastic syringe barrel comprising a plastic syringe barrel treated with plasma and coated with a polysiloxane-based lubricant coating treated with plasma, wherein each plasma treatment consisted essentially of uncharged energized gaseous species; and wherein the interior surface of the plasma-treated plastic syringe barrel with the plasma-treated polysiloxane-based lubricant coating has a surface density of ≤ 600 particles per 12 cm², ≤ 500 particles per 12 cm², or ≤ 400 particles per 12 cm², wherein the particles are greater than 8 microns in diameter. The presently disclosed subject matter describes a plastic syringe barrel comprising a plastic syringe barrel treated with plasma and coated with a polysiloxane-based lubricant coating treated with plasma, wherein each plasma is a downstream plasma generated at atmospheric pressure; and wherein the interior surface of the plasma-treated plastic syringe barrel with the plasma-treated polysiloxane-based lubricant coating has a surface density of ≤ 600 particles per 12 cm², ≤ 500 particles per 12 cm², or ≤ 400 particles per 12 cm², wherein the particles are greater than 8 microns in diameter.

The presently disclosed subject matter describes a plastic syringe barrel comprising a plastic syringe barrel treated with plasma and coated with a polysiloxane-based lubricant coating treated with plasma, wherein each plasma treatment consisted essentially of uncharged energized gaseous species; and wherein the interior surface of the plasma-treated plastic syringe barrel with the plasma-treated polysiloxane-based lubricant coating has a surface density of ≤ 100 particles per cm², ≤ 90 particles per cm², ≤ 50 particles per cm², ≤ 40 particles per cm², ≤ 35 particles per cm², or ≤ 30 particles per cm² wherein the particles are greater than 8 microns in diameter. The presently disclosed subject matter describes a plastic syringe barrel comprising a plastic syringe barrel treated with plasma and coated with a polysiloxane-based lubricant coating treated with plasma, wherein each plasma treatment comprises a downstream plasma generated at atmospheric pressure; and wherein the interior surface of the plasma-treated plastic syringe barrel with the plasma-treated polysiloxane-based lubricant coating has a surface density of ≤ 100 particles per cm², ≤ 90 particles per cm², ≤ 50 particles per cm², ≤ 40 particles per cm², ≤ 35 particles per cm², or ≤ 30 particles per cm² wherein the particles are greater than 8 microns in diameter.

The presently disclosed subject matter describes a syringe comprising a plastic syringe barrel described herein, a plunger rod, a plunger stopper, and a needle. The presently disclosed subject matter describes a syringe comprising a plastic syringe barrel described herein, a luer lock tip or slip tip, a plunger rod, and a plunger stopper. In some embodiments, the polysiloxane-based lubricant coating is a silicone oil coating. In some embodiments, the plastic syringe barrel comprises about 0.005mg/cm² to about 0.5mg/cm² of silicone oil. In some embodiments, the polysiloxane-based lubricant coating is a polydimethylsiloxane coating.

The presently disclosed subject matter describes a syringe comprises a plastic syringe barrel as described herein that contains a solution. In some embodiments, the particle level in the solution is ≤ 50 particles per ml for any particles ≥ 10µm in diameter or ≤ 5 particles per ml for any particles ≥ 25µm in diameter. In some embodiments, the syringe comprises a plastic syringe barrel that contains a solution comprising an anticoagulant, vaccine, or recombinant protein. In some embodiments, the syringe comprises a plastic syringe barrel that contains an anti-VEGF protein solution. In some embodiments, the syringe comprises a plastic syringe barrel that contains a solution comprising pegaptanib, ranibizumab, aflibercept, or bevacizumab. In some embodiments, the syringe comprises a plastic syringe barrel that contains an ophthalmic solution. In some embodiments, the particle level in the ophthalmic solution is ≤ 50 particles per ml for any particles ≥ 10µm in diameter or ≤ 5 particles per ml for any particles ≥ 25µm in diameter. In some embodiments, the plastic syringe barrel has a maximum fill volume of 1.0ml, 0.5ml, 0.3ml, 0.25ml, 0.10ml, or 0.05ml.

The presently disclosed subject matter describes a method of treating the eye, comprising intravitreally administering a solution or an ophthalmic solution to an eye with a syringe described herein.

The presently disclosed subject matter describes a method of producing a plastic syringe barrel with a stable silicone oil layer comprising providing a plastic syringe barrel, exposing an interior surface of the plastic syringe barrel to a plasma consisting essentially of uncharged energized gaseous species for 0.1 - 10 seconds; applying 0.005 - 0.5mg/cm² of silicone oil to the plasma-treated interior surface of the plastic syringe barrel to form a uniform silicone oil coating; and exposing the uniform silicone oil coating to a plasma consisting essentially of uncharged energized gaseous species for 0.1 - 10 seconds; wherein the interior surface of the plasma-treated plastic syringe barrel with plasma-treated silicone oil coating has a surface density of ≤ 600 particles per 12 cm², ≤ 500 particles per 12 cm², or ≤ 400 particles per 12 cm², wherein the particles are greater than 8 microns in diameter. The presently disclosed subject matter describes a method of producing a plastic syringe barrel with a stable silicone oil layer comprising providing a plastic syringe barrel, exposing an interior surface of the plastic syringe barrel to a downstream plasma generated at atmospheric pressure for 0.1 - 10 seconds; applying 0.005 - 0.5mg/cm² of silicone oil to the plasma-treated interior surface of the plastic syringe barrel to form a uniform silicone oil coating; and exposing the uniform silicone oil coating to a downstream plasma generated at atmospheric pressure for 0.1 - 10 seconds; wherein the interior surface of the plasma-treated plastic syringe barrel with plasma-treated silicone oil coating has a surface density of ≤ 600 particles per 12 cm², ≤ 500 particles per 12 cm², or ≤ 400 particles per 12 cm², wherein the particles are greater than 8 microns in diameter. In some embodiments, the method further comprises waiting at least 10 minutes, 20 minutes, 30 minutes, 40 minutes, 1 hour, 2 hours, or 3 hours before exposing the uniform silicone oil coating to the plasma.

The presently disclosed subject matter describes a method of producing a plastic syringe barrel with a stable silicone oil layer comprising providing a plastic syringe barrel, exposing an interior surface of the plastic syringe barrel to a plasma consisting essentially of uncharged energized gaseous species for 0.1 - 10 seconds; applying 0.005 - 0.5mg/cm² of silicone oil to the plasma-treated interior surface of the plastic syringe barrel to form a uniform silicone oil coating; and exposing the uniform silicone oil coating to a plasma consisting essentially of uncharged energized gaseous species for 0.1 - 10 seconds; wherein the interior surface of the plasma-treated plastic syringe barrel with plasma-treated silicone oil coating has a surface density of ≤ 100 particles per cm², ≤ 90 particles per cm², ≤ 50 particles per cm², ≤ 40 particles per cm². ≤ 35 particles per cm², or ≤ 30 particles per cm², wherein the particles are greater than 8 microns in diameter. The presently disclosed subject matter describes a method of producing a plastic syringe barrel with a stable silicone oil layer comprising providing a plastic syringe barrel, exposing an interior surface of the plastic syringe barrel to a downstream plasma generated at atmospheric pressure for 0.1 - 10 seconds; applying 0.005 - 0.5mg/cm² of silicone oil to the plasma-treated interior surface of the plastic syringe barrel to form a uniform silicone oil coating; and exposing the uniform silicone oil coating to a downstream plasma generated at atmospheric pressure for 0.1 - 10 seconds; wherein the interior surface of the plasma-treated plastic syringe barrel with plasma-treated silicone oil coating has a surface density of ≤ 100 particles per cm², ≤ 90 particles per cm², ≤ 50 particles per cm², ≤ 40 particles per cm², ≤ 35 particles per cm², or ≤ 30 particles per cm², wherein the particles are greater than 8 microns in diameter. In some embodiments, the method further comprises waiting at least 10 minutes, 20 minutes, 30 minutes, 40 minutes, 1 hour, 2 hours, or 3 hours before exposing the uniform silicone oil coating to the plasma.

The presently disclosed subject matter describes a method of producing a plastic syringe barrel with a stable silicone oil layer comprising providing a plastic syringe barrel, exposing an interior surface of the plastic syringe barrel to a plasma consisting essentially of uncharged energized gaseous species for 0.1- 10 seconds; applying 0.005 - 0.5mg/cm² of silicone oil to the plasma-treated interior surface of the plastic syringe barrel to form a uniform silicone oil coating; and exposing the uniform silicone oil coating to a plasma consisting essentially of uncharged energized gaseous species for 0.1 - 10sec; wherein the interior surface of the plasma-treated plastic syringe barrel with the plasma-treated silicone oil coating has at least a 75%, 80%, 85%, 90%, or 95% reduction in the number of particles greater than 8 microns in diameter as compared to an interior surface of a plastic syringe barrel with a silicone oil coating. The presently disclosed subject matter describes a method of producing a plastic syringe barrel with a stable silicone oil layer comprising providing a plastic syringe barrel, exposing an interior surface of the plastic syringe barrel to a downstream plasma generated at atmospheric pressure for 0.1- 10 seconds; applying 0.005 - 0.5mg/cm² of silicone oil to the plasma-treated interior surface of the plastic syringe barrel to form a uniform silicone oil coating; and exposing the uniform silicone oil coating to a downstream plasma generated at atmospheric pressure for 0.1 - 10sec; wherein the interior surface of the plasma-treated plastic syringe barrel with the plasma-treated silicone oil coating has at least a 75%, 80%, 85%, 90%, or 95% reduction in the number of particles greater than 8 microns in diameter as compared to an interior surface of a plastic syringe barrel with a silicone oil coating. In some embodiments, the method further comprises waiting at least 10 minutes, 20 minutes, 30 minutes, 40 minutes, 1 hour, 2 hours, or 3 hours before exposing the uniform silicone oil coating to the plasma.

In some embodiments, the uncharged energized gaseous species comprises excited argon gas atoms. In some embodiments, the plastic is a cyclic olefin polymer (COP), cyclic olefin copolymer (COC), polyethylene (PE), polycarbonate (PC), polypropylene (PP), or polyethylene terephthalate (PET). In some embodiments, the plastic syringe has a maximum fill volume of 1.0ml, 0.5ml, 0.3ml, 0.25ml, 0.10ml, or 0.05ml.

The presently disclosed subject matter describes a method of producing a syringe with a plastic syringe barrel with a stable polysiloxane-based lubricant coating comprising a method of producing the plastic syringe barrel as described herein, and assembling the plastic syringe barrel with a plunger rod, plunger stopper, and needle. In some embodiments, the plastic syringe barrel contains a solution and wherein the particle level in the solution is ≤ 50 particles per ml for any particles ≥ 10µm in diameter or ≤ 5 particles per ml for any particles ≥ 25µm in diameter.

The presently disclosed subject matter describes a plastic syringe comprising a plastic barrel, a plunger rod, a plunger stopper, wherein the plastic barrel has a stable silicone oil coating and is produced by providing a plastic syringe barrel, exposing an interior surface of the plastic syringe barrel to a plasma consisting essentially of uncharged energized gaseous species for 0.1- 10 seconds; applying 0.005 - 0.5 mg/cm² of silicone oil to the plasma-treated interior surface of the plastic syringe barrel to form a uniform silicone oil coating; and exposing the uniform silicone oil coating to a plasma consisting essentially of uncharged energized gaseous species; wherein the interior surface of the plasma-treated plastic syringe barrel with the plasma-treated silicone oil coating has a surface density of ≤ 600 particles per 12 cm², ≤ 500 particles per 12 cm², or ≤ 400 particles per 12 cm², wherein the particles are greater than 8 microns in diameter. The presently disclosed subject matter describes a plastic syringe comprising a plastic barrel, a plunger rod, a plunger stopper, wherein the plastic barrel has a stable silicone oil coating and is produced by providing a plastic syringe barrel, exposing an interior surface of the plastic syringe barrel to a downstream plasma generated at atmospheric pressure for 0.1- 10 seconds; applying 0.005 - 0.5 mg/cm² of silicone oil to the plasma-treated interior surface of the plastic syringe barrel to form a uniform silicone oil coating; and exposing the uniform silicone oil coating to a downstream plasma generated at atmospheric pressure; wherein the interior surface of the plasma-treated plastic syringe barrel with the plasma-treated silicone oil coating has a surface density of ≤ 600 particles per 12 cm², ≤ 500 particles per 12 cm², or ≤ 400 particles per 12 cm², wherein the particles are greater than 8 microns in diameter.

The presently disclosed subject matter describes a plastic syringe comprising a plastic barrel, a plunger rod, a plunger stopper, wherein the plastic barrel has a stable silicone oil coating and is produced by providing a plastic syringe barrel, exposing an interior surface of the plastic syringe barrel to a plasma consisting essentially of uncharged energized gaseous species for 0.1- 10 seconds, applying 0.005 - 0.5 mg/cm² of silicone oil to the plasma-treated interior surface of the plastic syringe barrel to form a uniform silicone oil coating; and exposing the uniform silicone oil coating to a plasma consisting essentially of uncharged energized gaseous species; wherein the interior surface of the plasma-treated plastic syringe barrel with the plasma-treated silicone oil coating has a surface density of ≤ 100 particles per cm², ≤ 90 particles per cm², ≤ 50 particles per cm², ≤ 40 particles per cm², ≤ 35 particles per cm², or ≤ 30 particles per cm² wherein the particles are greater than 8 microns in diameter. The presently disclosed subject matter describes a plastic syringe comprising a plastic barrel, a plunger rod, a plunger stopper, wherein the plastic barrel has a stable silicone oil coating and is produced by providing a plastic syringe barrel, exposing an interior surface of the plastic syringe barrel to a downstream plasma generated at atmospheric pressure for 0.1- 10 seconds, applying 0.005 - 0.5 mg/cm² of silicone oil to the plasma-treated interior surface of the plastic syringe barrel to form a uniform silicone oil coating; and exposing the uniform silicone oil coating to a downstream plasma generated at atmospheric pressure; wherein the interior surface of the plasma-treated plastic syringe barrel with the plasma-treated silicone oil coating has a surface density of ≤ 100 particles per cm², ≤ 90 particles per cm², ≤ 50 particles per cm², ≤ 40 particles per cm², ≤ 35 particles per cm², or ≤ 30 particles per cm² wherein the particles are greater than 8 microns in diameter.

The presently disclosed subject matter describes a plastic syringe comprising a plastic syringe barrel, a plunger rod, a plunger stopper, wherein the plastic barrel has a stable silicone oil coating and is produced by providing a plastic syringe barrel, exposing an interior surface of the plastic syringe barrel to a plasma consisting essentially of uncharged energized gaseous species for 0.1- 10 seconds; applying 0.005 - 0.5mg/cm² of silicone oil to the plasma-treated interior surface of the plastic syringe barrel to form a uniform silicone oil coating; and exposing the uniform silicone oil coating to a plasma consisting essentially of uncharged energized gaseous species; wherein the interior surface of the plasma-treated plastic syringe barrel with the plasma-treated silicone oil coating has at least a 75%, 80%, 85%, 90%, or 95% reduction in the number of particles greater than 8 microns in diameter as compared to an interior surface of a plastic syringe barrel with a silicone oil coating. The presently disclosed subject matter describes a plastic syringe comprising a plastic syringe barrel, a plunger rod, a plunger stopper, wherein the plastic barrel has a stable silicone oil coating and is produced by providing a plastic syringe barrel, exposing an interior surface of the plastic syringe barrel to a downstream plasma generated at atmospheric pressure for 0.1- 10 seconds; applying 0.005 - 0.5mg/cm² of silicone oil to the plasma-treated interior surface of the plastic syringe barrel to form a uniform silicone oil coating; and exposing the uniform silicone oil coating to a downstream plasma generated at atmospheric pressure; wherein the interior surface of the plasma-treated plastic syringe barrel with the plasma-treated silicone oil coating has at least a 75%, 80%, 85%, 90%, or 95% reduction in the number of particles greater than 8 microns in diameter as compared to an interior surface of a plastic syringe barrel with a silicone oil coating.

In some embodiments, the plastic syringe comprises a plastic syringe barrel that contains a solution. In some embodiments, the particle level in the solution is ≤ 50 particles per ml for any particles ≥ 10µm in diameter or ≤ 5 particles per ml for any particles ≥ 25µm in diameter. In some embodiments, the plastic syringe comprises a plastic syringe barrel that contains a biologic. In some embodiments, the plastic syringe comprises a plastic syringe barrel that contains a solution comprising an anticoagulant, vaccine, or recombinant protein. In some embodiments, the plastic syringe syringe comprises a plastic syringe barrel that contains an anti-VEGF protein solution. In some embodiments, the plastic syringe comprises a plastic syringe barrel that contains a solution comprising pegaptanib, ranibizumab, aflibercept, or bevacizumab. In some embodiments, the plastic syringe comprises a plastic syringe barrel that contains an ophthalmic solution. In some embodiments, the particle level in the ophthalmic solution is ≤ 50 particles per ml for any particles ≥ 10µm in diameter or ≤ 5 particles per ml for any particles ≥ 25µm in diameter.

The presently disclosed subject matter describes a method of treating the eye, comprising intravitreally administering a solution or an ophthalmic solution to an eye with a syringe produced by a method described herein.

The presently syringe comprising a plastic syringe barrel treated with plasma and coated with a polysiloxane-based lubricant coating treated with plasma, wherein each plasma treatment consisted essentially of uncharged energized gaseous species, a plunger rod, a plunger stopper, and a needle; wherein the plastic syringe barrel contains a solution and the particle level in the solution is ≤ 50 particles per ml for any particles ≥ 10µm in diameter or ≤ 5 particles per ml for any particles ≥ 25µm in diameter. The presently disclosed subject matter describes a method of treating the eye, comprising intravitreally administering the solution to an eye with the syringe. A syringe comprising a plastic syringe barrel treated with plasma and coated with a polysiloxane-based lubricant coating treated with plasma, wherein each plasma treatment is a downstream plasma generated at atmospheric pressure, a plunger rod, a plunger stopper, and a needle; wherein the plastic syringe barrel contains a solution and the particle level in the solution is ≤ 50 particles per ml for any particles ≥ 10µm in diameter or ≤ 5 particles per ml for any particles ≥ 25µm in diameter. The presently disclosed subject matter describes a method of treating the eye, comprising intravitreally administering the solution to an eye with the syringe. In some embodiments, the solution is an anti-VEGF protein solution. In some embodiments, the solution comprises an anticoagulant, vaccine, or recombinant protein. In some embodiments, the solution is an ophthalmic solution. In some embodiments, the solution comprises pegaptanib, ranibizumab, aflibercept, or bevacizumab. The presently disclosed subject matter describes syringe comprising a plastic syringe barrel treated with plasma and coated with a polysiloxane-based lubricant coating treated with plasma, wherein each plasma treatment consisted essentially of uncharged energized gaseous species, a luer lock tip or slip tip, a plunger rod, and a plunger stopper; wherein the plastic syringe barrel contains a solution and the particle level in the solution is ≤ 50 particles per ml for any particles ≥ 10µm in diameter or ≤ 5 particles per ml for any particles ≥ 25µm in diameter. The presently disclosed subject matter describes syringe comprising a plastic syringe barrel treated with plasma and coated with a polysiloxane-based lubricant coating treated with plasma, wherein each plasma treatment is a downstream plasma generated at atmospheric pressure, a luer lock tip or slip tip, a plunger rod, and a plunger stopper; wherein the plastic syringe barrel contains a solution and the particle level in the solution is ≤ 50 particles per ml for any particles ≥ 10µm in diameter or ≤ 5 particles per ml for any particles ≥ 25µm in diameter.

The presently disclosed subject matter describes a plastic syringe barrel comprising a plastic syringe barrel treated with plasma and coated with a perfluoropolyether lubricant coating treated with plasma, wherein each plasma treatment consisted essentially of uncharged energized gaseous species or each plasma treatment was a downstream plasma; and wherein the interior surface of the plasma-treated plastic syringe barrel with the plasma-treated perfluoropolyether lubricant coating has at least a 75%, 80%, or 95% reduction in the number of particles greater than 8 microns in diameter as compared to an interior surface of a plastic syringe barrel with a perfluoropolyether lubricant coating.

The presently disclosed subject matter describes a plastic syringe barrel comprising a plastic syringe barrel treated with plasma and coated with a perfluoropolyether lubricant coating treated with plasma, wherein each plasma treatment consisted essentially of uncharged energized gaseous species or each plasma treatment comprised a downstream plasma; and wherein the interior surface of the plasma-treated plastic syringe barrel with the plasma-treated perfluoropolyether lubricant coating has a surface density of ≤ 600 particles per 12 cm², ≤ 500 particles per 12 cm², or ≤ 400 particles per 12 cm², wherein the particles are greater than 8 microns in diameter.

The presently disclosed subject matter describes plastic syringe barrel comprising a plastic syringe barrel treated with plasma and coated with a perfluoropolyether lubricant coating treated with plasma, wherein each plasma treatment consisted essentially of uncharged energized gaseous species or each plasma treatment comprised a downstream plasma; and wherein the interior surface of the plasma-treated plastic syringe barrel with the plasma-treated perfluoropolyether lubricant coating has a surface density of ≤ 100 particles per cm², ≤ 90 particles per cm², ≤ 50 particles per cm², ≤ 40 particles per cm², ≤ 35 particles per cm², or ≤ 30 particles per cm², wherein the particles are greater than 8 microns in diameter.

The presently disclosed subject matter describes a syringe comprising a plastic syringe barrel described herein, a plunger rod, a plunger stopper, and a needle. The presently disclosed subject matter describes a syringe comprising a plastic syringe barrel described herein, a plunger rod, a plunger stopper, and a luer lock tip or slip tip. In some embodiments, the plastic syringe barrel contains a solution. In some embodiments, the particle level in the solution is ≤ 50 particles per ml for any particles ≥ 10µm in diameter or ≤ 5 particles per ml for any particles ≥ 25µm in diameter. In some embodiments, the plastic syringe barrel contains a solution comprising an anticoagulant, vaccine, or recombinant protein. In some embodiments, the plastic syringe barrel contains an anti-VEGF protein solution comprising pegaptanib, ranibizumab, aflibercept, or bevacizumab. In some embodiments, the plastic syringe barrel contains an ophthalmic solution. In some embodiments, the particle level in the ophthalmic solution is ≤ 50 particles per ml for any particles ≥ 10µm in diameter or ≤ 5 particles per ml for any particles ≥ 25µm in diameter. The presently disclosed subject matter describes a method of treating the eye, comprising intravitreally administering the solution or the ophthalmic solution to an eye with a syringe described herein.

The presently disclosed subject matter describes a method of producing a plastic syringe barrel with a stable lubricant layer comprising providing a plastic syringe barrel, exposing an interior surface of the plastic syringe barrel to a plasma consisting essentially of uncharged energized gaseous species for 0.1 - 10 seconds; applying 0.005 - 0.5mg/cm² of perfluoropolyether to the plasma-treated interior surface of the plastic syringe barrel to form a uniform perfluoropolyether coating; and exposing the uniform perfluoropolyether coating to a plasma consisting essentially of uncharged energized gaseous species for 0.1 - 10 seconds; wherein the interior surface of the plasma-treated plastic syringe barrel with plasma-treated perfluoropolyether coating has a surface density of ≤ 600 particles per 12 cm², ≤ 500 particles per 12 cm², ≤ 400 particles per 12 cm², ≤ 100 particles per cm², ≤ 90 particles per cm², ≤ 50 particles per cm², ≤ 40 particles per cm². ≤ 35 particles per cm², or ≤ 30 particles per cm², wherein the particles are greater than 8 microns in diameter.

The presently disclosed subject matter describes a syringe comprising a plastic syringe barrel treated with plasma and coated with a perfluoropolyether lubricant coating treated with plasma, wherein each plasma treatment consisted essentially of uncharged energized gaseous species, a plunger rod, a plunger stopper, and a needle: wherein the plastic syringe barrel contains a solution and the particle level in the solution is ≤ 50 particles per ml for any particles ≥ 10µm in diameter or ≤ 5 particles per ml for any particles ≥ 25µm in diameter. The presently disclosed subject matter describes a method of treating the eye, comprising intravitreally administering the solution to an eye with a syringe described herein. In some embodiments, the solution comprises an anticoagulant, vaccine, or recombinant protein, wherein the solution is an ophthalmic solution, wherein the solution is an anti-VEGF protein solution comprising pegaptanib, ranibizumab, aflibercept, or bevacizumab.

The presently disclosed subject matter describes a syringe comprising a plastic syringe barrel treated with plasma and coated with a perfluoropolyether lubricant coating treated with plasma, wherein each plasma treatment consisted essentially of uncharged energized gaseous species, a luer lock tip or slip tip, a plunger rod, and a plunger stopper; wherein the plastic syringe barrel contains a solution and the particle level in the solution is ≤ 50 particles per ml for any particles ≥ 10µm in diameter or ≤ 5 particles per ml for any particles ≥ 25µm in diameter.

### BRIEF DESCRIPTION OF THE DRAWINGS

The accompanying drawings, which are incorporated herein and form part of the specification, illustrate various embodiments of the present invention and, together with the description, further serve to explain the principles of the invention and to enable a person skilled in the pertinent art to make and use the invention. In the drawings, like reference numbers indicate identical or functionally similar elements.

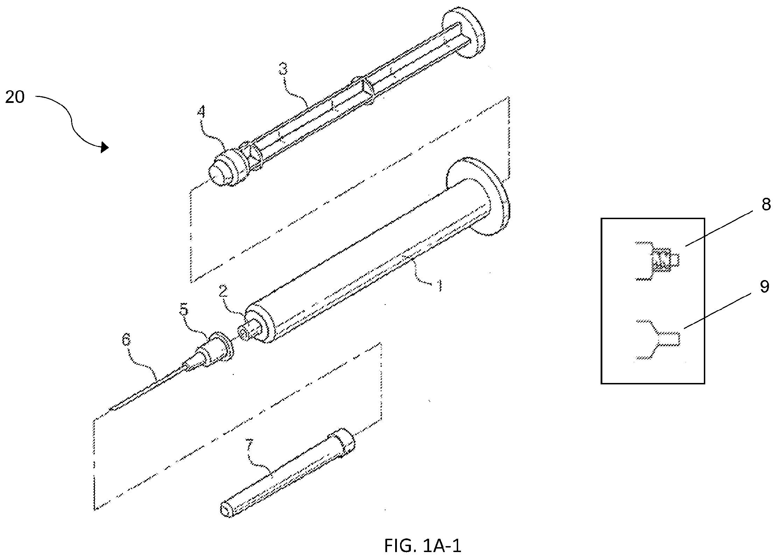

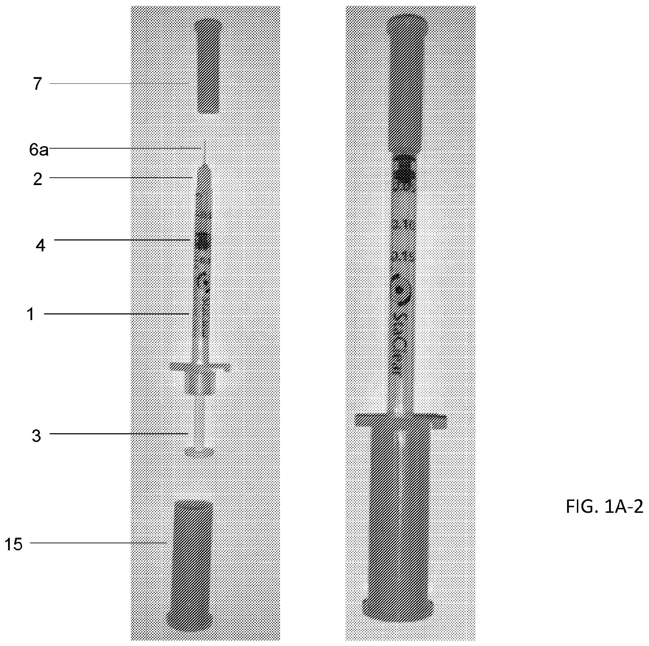

FIG. 1A is an exploded perspective view of a syringe (left) and inset images of a luer slip tip (top) and luer lock tip (bottom).

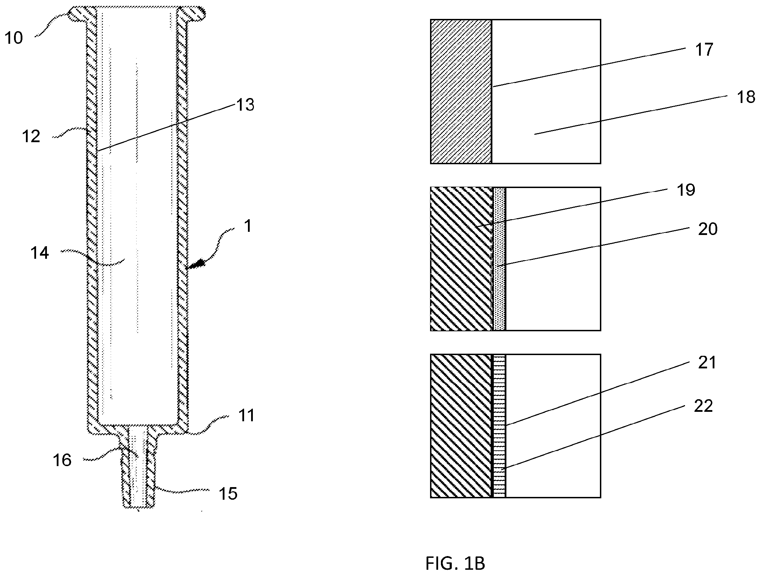

FIG. 1B is cross-section view of a plastic syringe barrel and syringe tip (left), and cross-section diagrams to illustrate the various surfaces (right)
FIG. 1C-1 are brightfield images of the interior surface of an empty/unfilled COP syringe barrel without any coating.
FIG. 1C-2 are corresponding darkfield images of the interior surface of the empty/unfilled COP syringe barrel without any coating (left), and an enlarged view of a portion of those darkfield images (right).
FIG. 1D-1 are brightfield images of the interior surface of an empty/unfilledCOP syringe barrel immediately after spray of 1000cSt silicone oil.

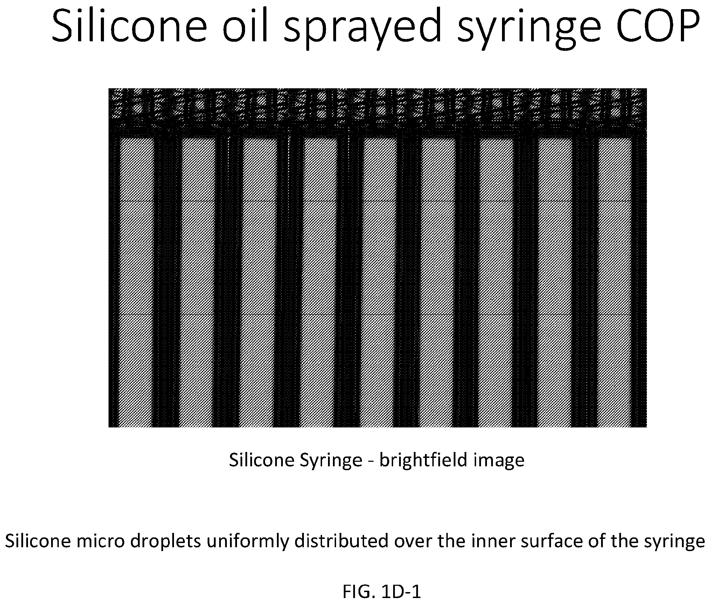

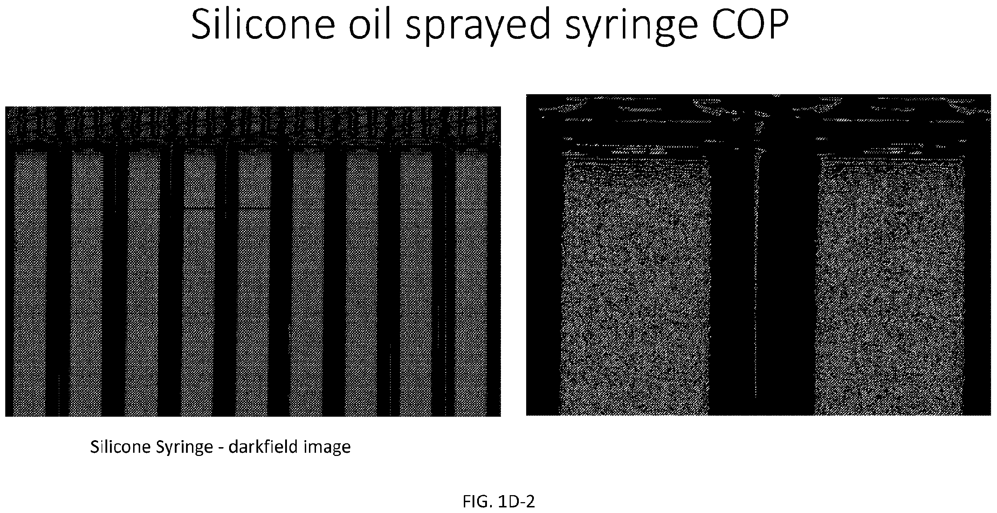

FIG. 1D-2 are corresponding darkfield images of the interior surface of the empty/unfilled COP syringe barrel with sprayed on silicone oil (left), and an enlarged view of a portion of those darkfield images (right).

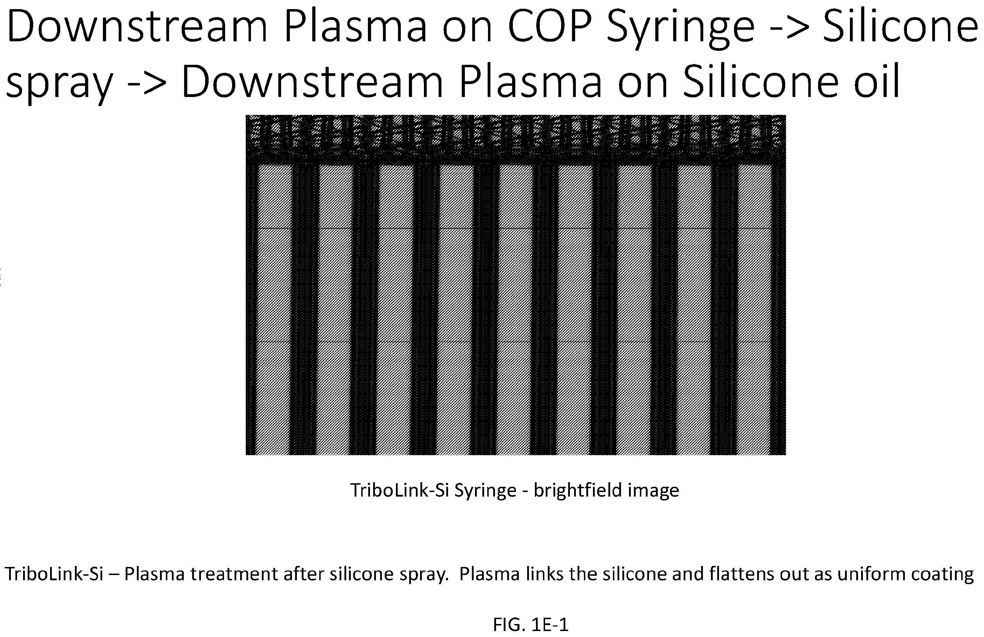

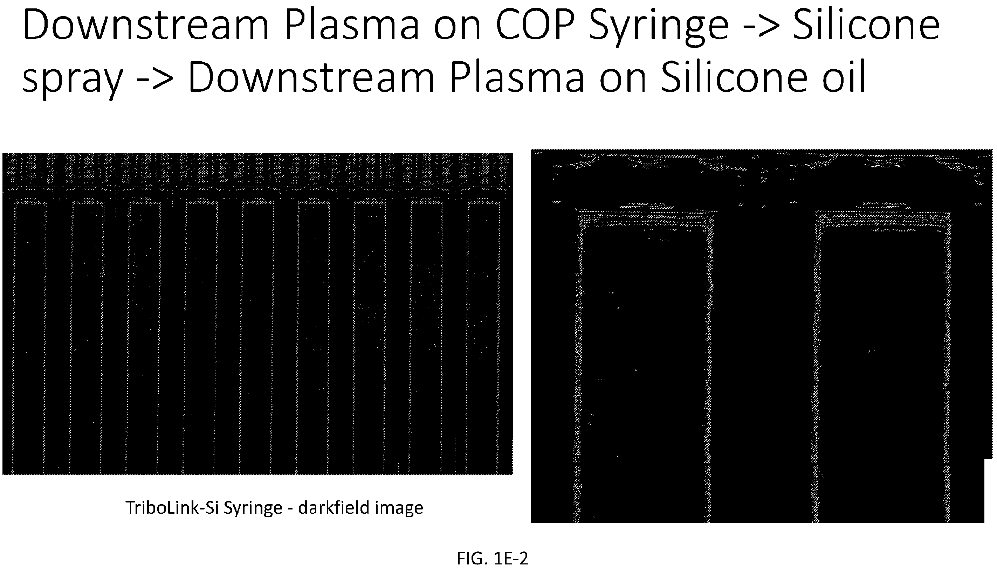

FIG. 1E-1 are brightfield images of the interior surface of an empty/unfilled plasma-treated COP syringe barrel with plasma-treated silicone oil according to an embodiment of the invention. The plasma treatment of the COP syringe barrel and the plasma treatment of the silicone oil were each a plasma treatment consisting essentially of uncharged energized gaseous species.
FIG. 1E-2 are corresponding darkfield images of the interior surface of the empty/unfilled plasma-treated COP syringe barrel with plasma-treated silicone oil according to an embodiment of the invention (left), and an enlarged view of a portion of those darkfield images (right). The plasma treatment of the COP syringe barrel and the plasma treatment of the silicone oil were each a plasma treatment consisting essentially of uncharged energized gaseous species.

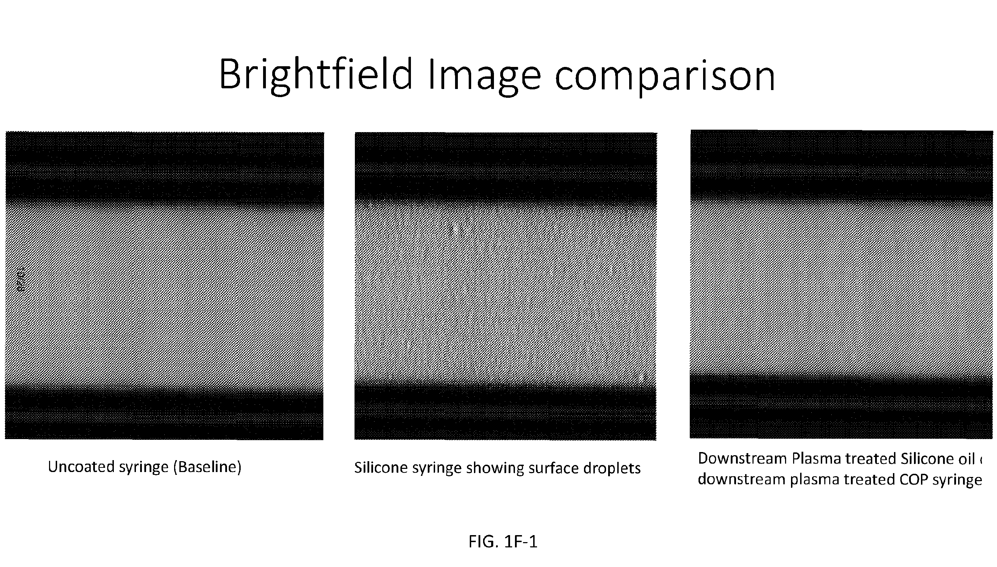

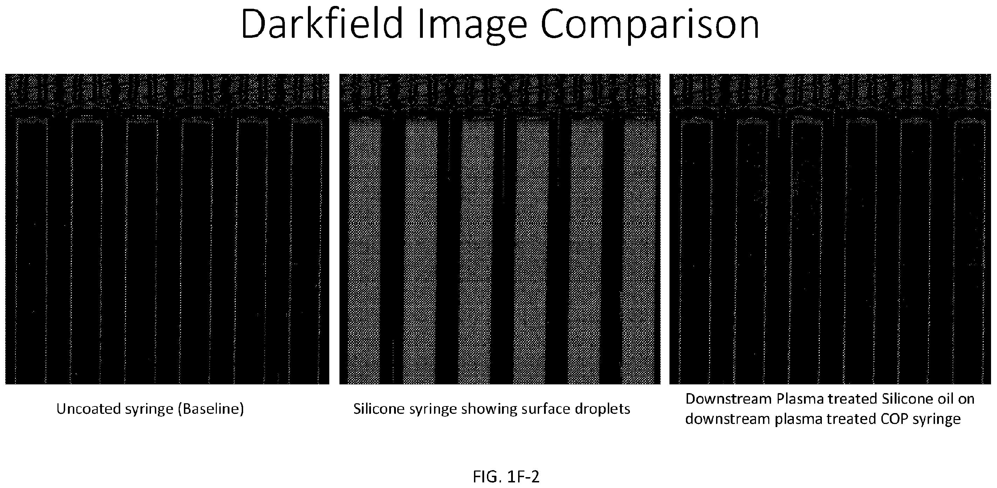

FIG. 1F-1 are enlarged brightfield images of the interior surface of the empty/unfilled COP syringe barrel without any coating (left), the interior surface of the empty/unfilled COP syringe barrel with sprayed on silicone oil (middle), and the interior surface of the empty/unfilled plasma-treated COP syringe with plasma-treated silicone oil according to an embodiment of the invention, wherein the plasma treatment of the COP syringe barrel and the plasma treatment of the silicone oil were each a plasma treatment consisting essentially of uncharged energized gaseous species. (right).
FIG. 1F-2 are enlarged darkfield images of the interior surface of the empty/unfilled COP syringe barrel without any coating (left), the interior surface of the empty/unfilled COP syringe barrel with sprayed on silicone oil (middle), and the interior surface of the empty/unfilled plasma-treated COP syringe with plasma-treated silicone oil according to an embodiment of the invention, wherein the plasma treatment of the COP syringe barrel and the plasma treatment of the silicone oil were each a plasma treatment consisting essentially of uncharged energized gaseous species. (right).
FIG. G-1 are brightfield images of the interior surface of an empty/unfilled downstream plasma-treated COP syringe barrel with sprayed-on silicone oil.
FIG. G-2 are corresponding darkfield images of the interior surface of the empty/unfilled downstream plasma-treated COP syringe barrel with sprayed-on silicone oil (left), and an enlarged view of a portion of those darkfield images (right).

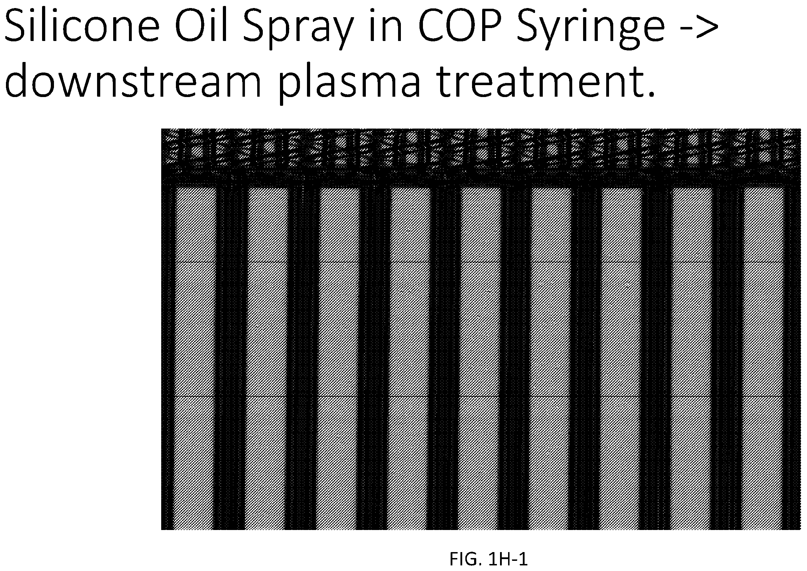

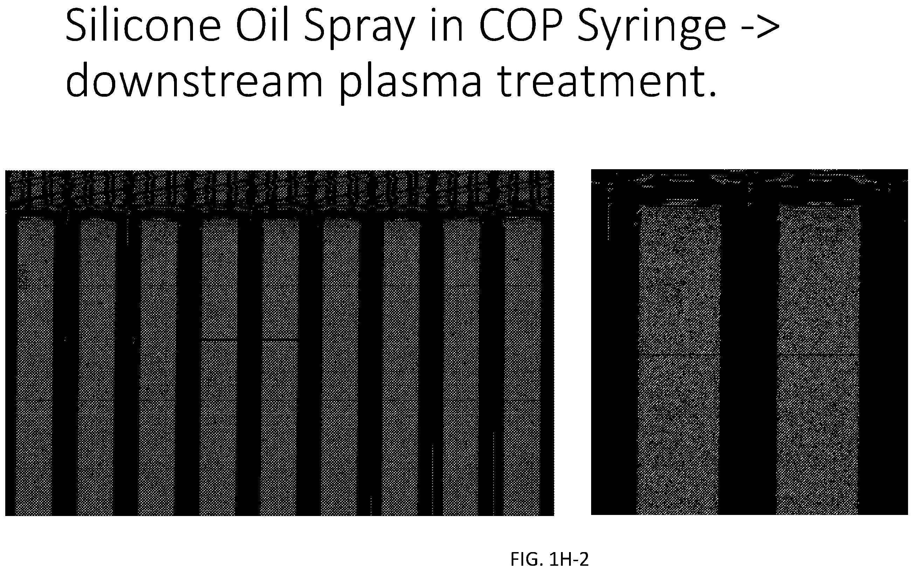

FIG. 1H-1 are brightfield images of the interior surface of an empty/unfilled COP syringe barrel with downstream plasma-treated silicone oil.
FIG. 1H-2 are corresponding darkfield images of the empty/unfilled interior surface of the COP syringe barrel with downstream plasma-treated silicone oil (left), and an enlarged view of a portion of those darkfield images (right).

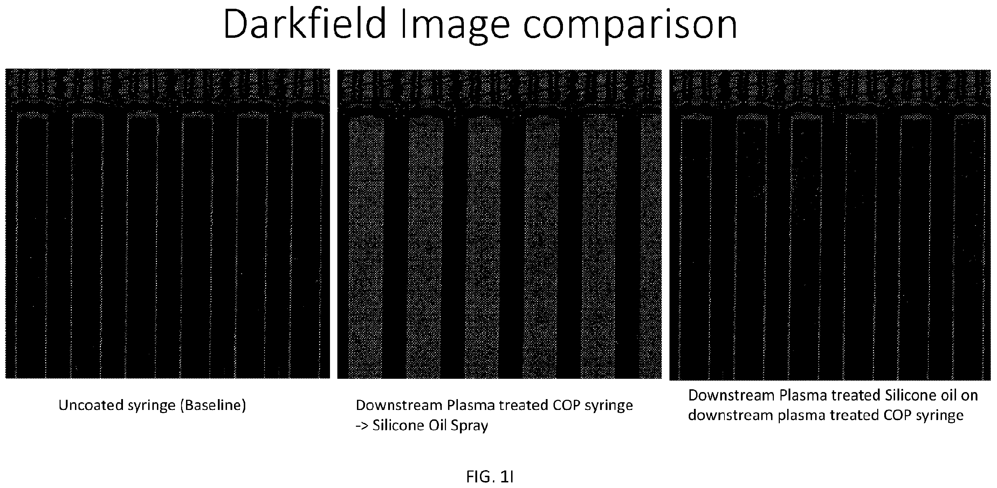

FIG. 1I are darkfield images of the interior surface of the empty/unfilled COP syringe barrel without any coating (left), the interior surface of the empty/unfilled downstream plasma-treated COP syringe barrel with sprayed-on silicone oil (middle), and the interior surface of the empty/unfilled plasma-treated COP syringe with plasma-treated silicone oil according to an embodiment of the invention, wherein the plasma treatment of the COP syringe barrel and the plasma treatment of the silicone oil were each a plasma treatment consisting essentially of uncharged energized gaseous species (right).

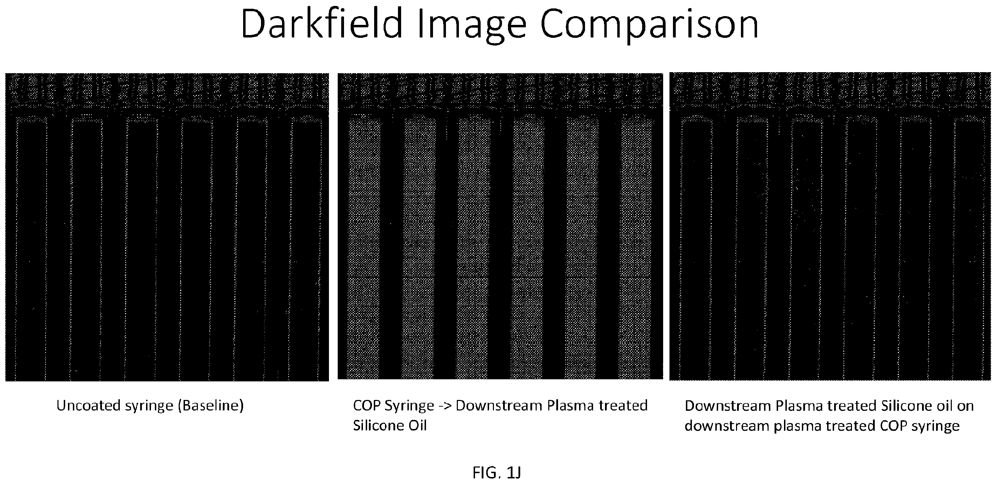

FIG. 1J are darkfield images of the interior surface of the empty/unfilled COP syringe barrel without any coating (left), the interior surface of the empty/unfilled COP syringe barrel with downstream plasma-treated silicone oil (middle), and the interior surface of the empty/unfilled plasma-treated COP syringe with plasma-treated silicone oil according to an embodiment of the invention, wherein the plasma treatment of the COP syringe barrel and the plasma treatment of the silicone oil were each a plasma treatment consisting essentially of uncharged energized gaseous species. (right).

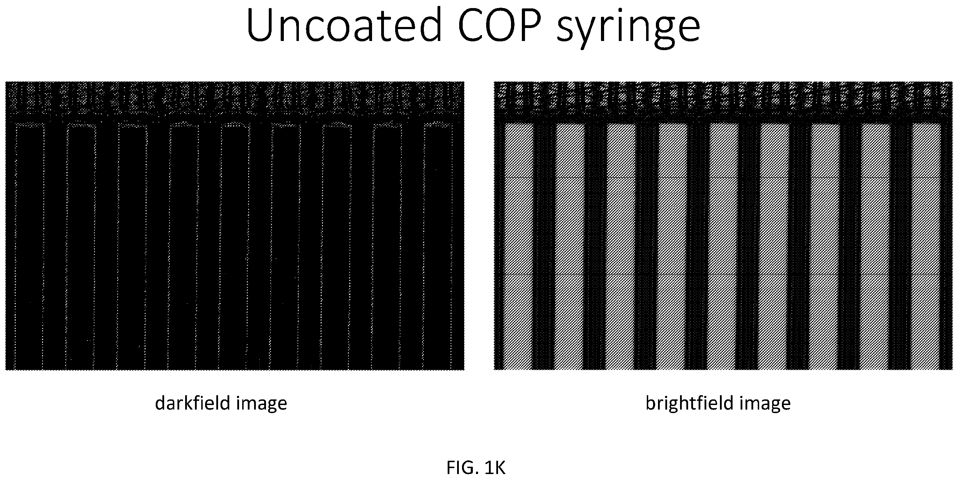

FIG. 1K are darkfield images (left) and brightfield images (right) of the interior surface of an empty/unfilled COP syringe barrel without any coating.

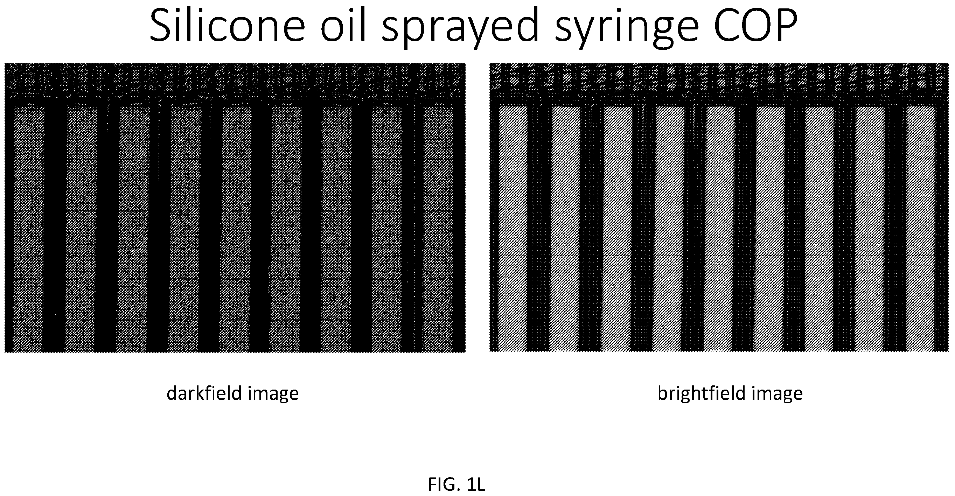

FIG. 1L are darkfield images (left) and brightfield images (right) of the interior surface of an empty/unfilled COP syringe barrel immediately after spray of 1000cSt silicone oil.

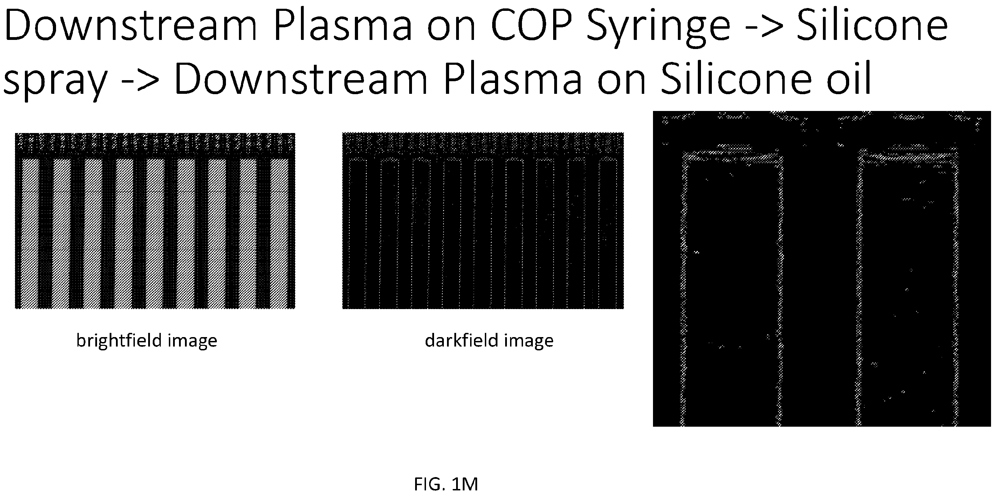

FIG. 1M are brightfield images (left), darkfield images (middle) of the interior surface of an empty/unfilled plasma-treated COP syringe barrel with plasma-treated silicone oil according to an embodiment of the invention (middle), and an enlarged view of a portion of those darkfield images (right). The plasma treatment of the COP syringe barrel and the plasma treatment of the silicone oil were each a plasma treatment consisting essentially of uncharged energized gaseous species.

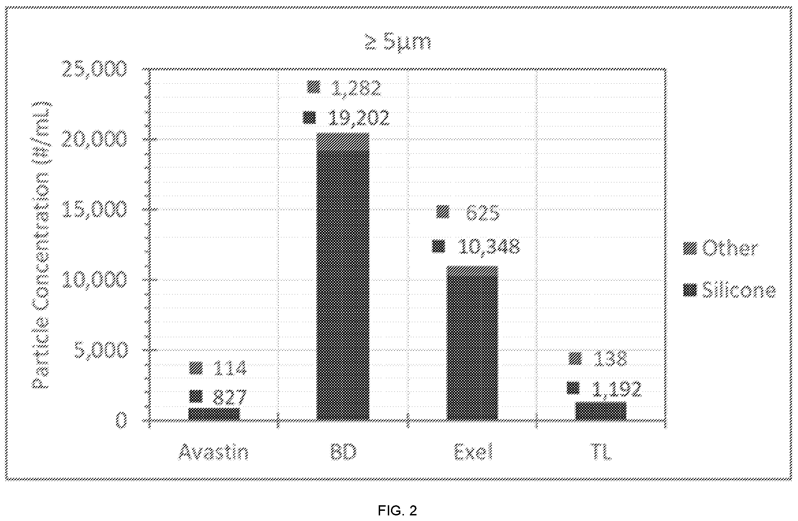

FIG. 2 is a bar graph comparing particle concentration (number of particles per mL) of particles ≥ 5µm between the following biologic solution sources: Avastin^{®} straight from the vial, BD U100 insulin syringes filled with Avastin^{®}, Exel U100 insulin syringes with Avastin^{®}, and general use 0.25ml syringe treated according to methods described herein and filled with Avastin^{®}.

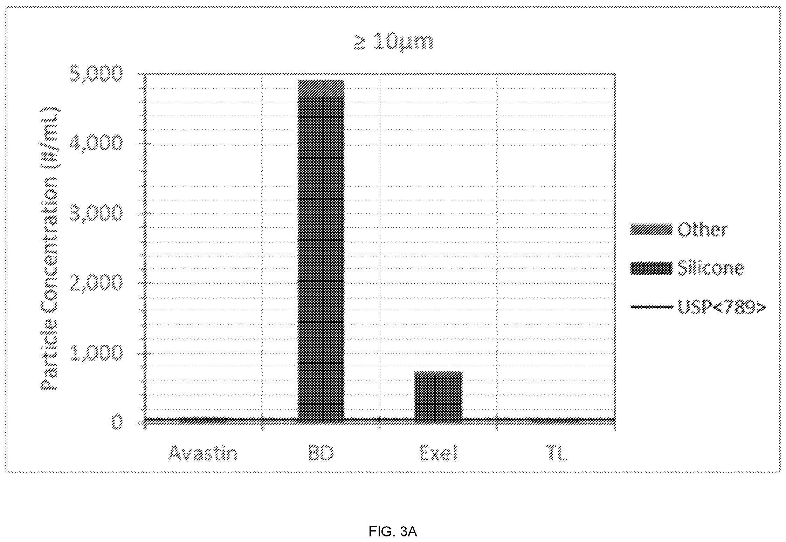

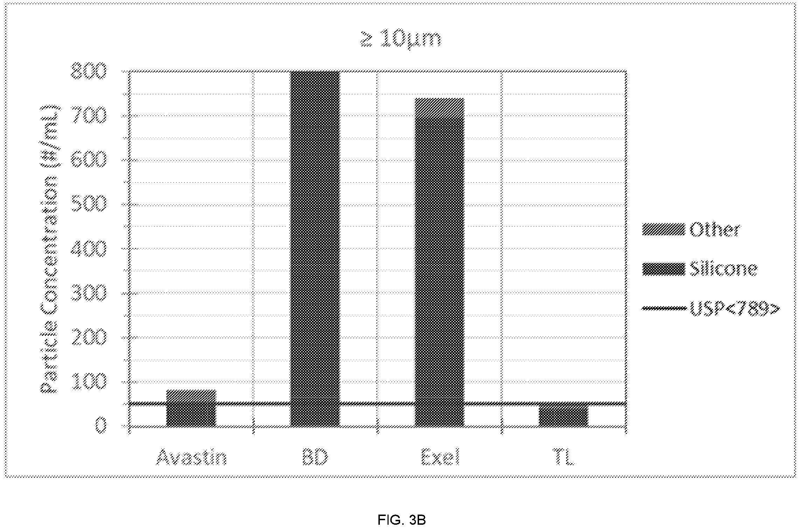

FIG. 3A a bar graph comparing particle concentration (number of particles per mL) of particles ≥ 10µm between the following biologic solution sources: Avastin^{®} straight from the vial, BD U100 insulin syringes filled with Avastin^{®}, Exel U100 insulin syringes with Avastin^{®}, and general use 0.25ml syringe treated according to the methods described herein and filled with Avastin^{®}. FIG 3B is an enlarged view of the bar graph in FIG. 3A.

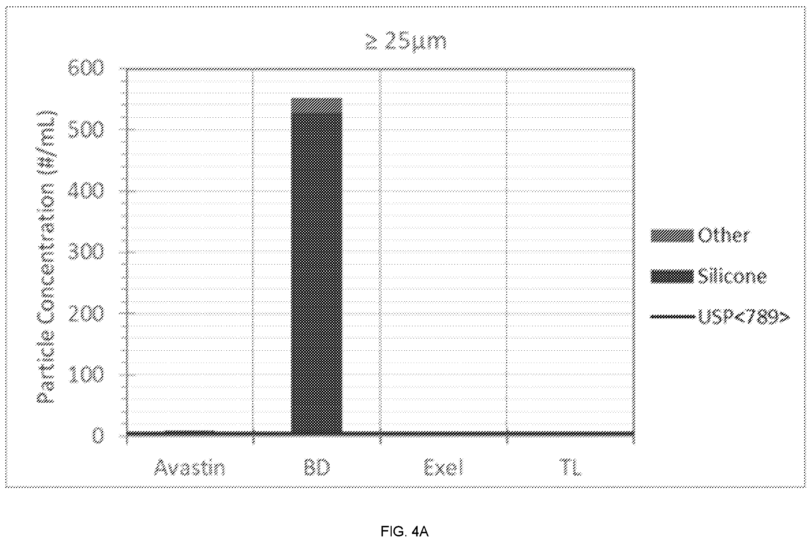

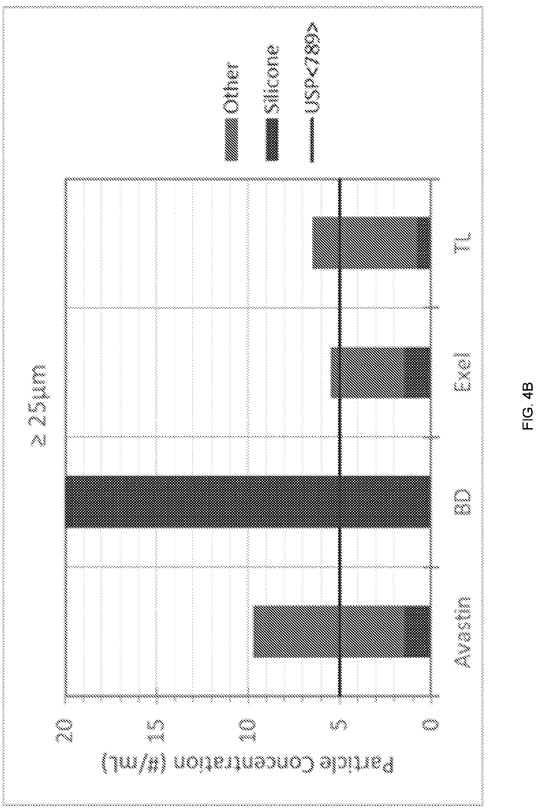

FIG. 4A a bar graph comparing particle concentration (number of particles per mL) of particles ≥ 25µm between the following biologic solution sources: Avastin^{®} straight from the vial, BD U100 insulin syringes filled with Avastin^{®}, Exel U100 insulin syringes with Avastin^{®}, and general use 0.25ml syringe treated according to methods described herein and filled with Avastin^{®}. FIG 4B is an enlarged view of the bar graph in FIG. 4A.

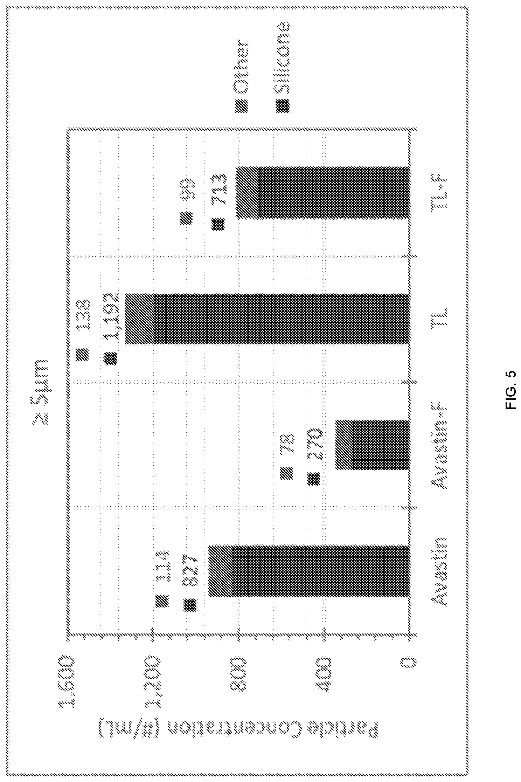

FIG. 5 a bar graph comparing particle concentration (number of particles per mL) of particles ≥ 5µm between the following biologic solution sources: Avastin^{®} straight from the vial, filtered Avastin^{®} straight from the vial, general use 0.25ml syringe treated according to methods described herein and filled with Avastin^{®}, and general use 0.25ml syringe treated according methods described herein and filled with filtered Avastin^{®}.

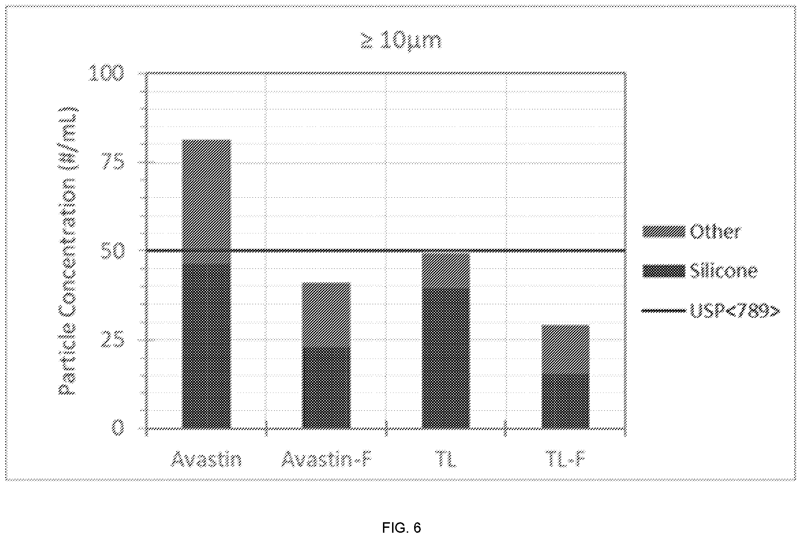

FIG. 6 a bar graph comparing particle concentration (number of particles per mL) of particles ≥ 10µm between the following biologic solution sources: Avastin^{®} straight from the vial, filtered Avastin^{®} straight from the vial, general use 0.25ml syringe treated according to the methods described herein and filled with Avastin^{®}, and general use 0.25ml syringe treated according to the methods described herein and filled with filtered Avastin^{®}.

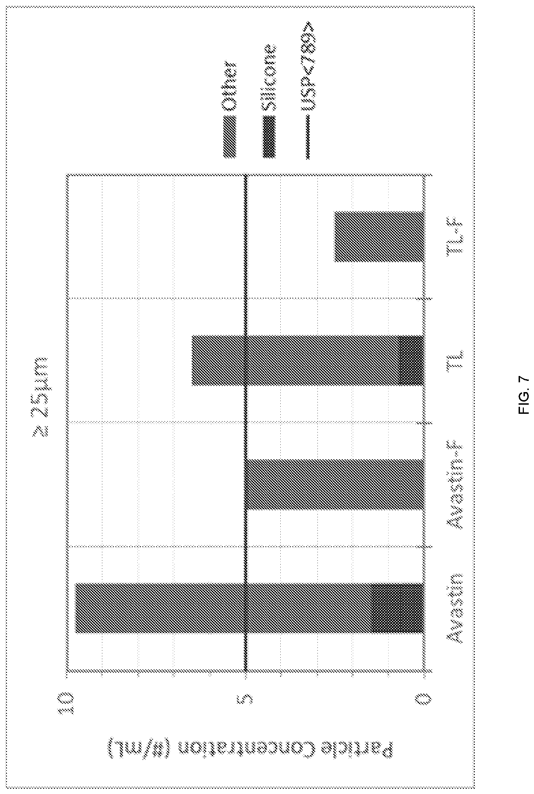

FIG. 7 a bar graph comparing particle concentration (number of particles per mL) of particles ≥ 25µm between the following biologic solution sources: Avastin^{®} straight from the vial, filtered Avastin^{®} straight from the vial, general use 0.25ml syringe treated according to the methods described herein and filled with Avastin^{®}, and general use 0.25ml syringe treated according to the methods described herein and filled with filtered Avastin^{®}.

### DETAILED DESCRIPTION OF EMBODIMENTS

While the present invention may be embodied in many different forms, a number of illustrative embodiments are described herein with the understanding that the present disclosure is to be considered as providing examples of the principles of the invention and such examples are not intended to limit the invention to preferred embodiments described herein and/or illustrated herein. The claimed subject matter might also be embodied in other ways, to include different steps or elements similar to the ones described in this document, in conjunction with other present or future technologies. Moreover, although the term "step" may be used herein to connote different aspects of methods employed, the term should not be interpreted as implying any particular order among or between various steps herein disclosed unless and except when the order of individual steps is explicitly described.

Embodiments of the present invention will now be described more fully hereinafter with reference to the accompanying drawings, in which some, but not all, embodiments of the invention are shown. Indeed, the invention may be embodied in many different forms and should not be construed as limited to the embodiments set forth herein; rather, these embodiments are provided so that this disclosure will satisfy applicable legal requirements. Like numbers refer to elements throughout. Other details of the embodiments of the invention should be readily apparent to one skilled in the art from the drawings. Although the invention has been described based upon these preferred embodiments, it would be apparent to those skilled in the art that certain modifications, variations, and alternative constructions would be apparent, while remaining within the spirit and scope of the invention.

The presently disclosed subject matter is now described in more detail.

FIG. 1A-1 is an exploded perspective view of an exemplary syringe **20** (left) and inset images of a luer slip tip (top) and luer lock tip (bottom). FIG. 1A illustrates a plastic syringe barrel **1,** a syringe tip **2,** a plunger rod **3** inserted into the plastic syringe barrel and movable forward or backward along the length of the plastic syringe barrel, a plunger stopper or seal **4** connected to the front of the plunger rod **3** and in airtight contact with a portion of the interior surface of the plastic syringe barrel as the plunger rod is moved forward or backward, a needle **6** and needle hub **5** that connects to the syringe tip **2,** and needle safety cap **7.** In other embodiments, the syringe **20** does not comprise a needle **6,** needle hub **5** connected to the syringe top **2,** and needle safety cap **7.** The inset images of FIG. 1A illustrate two configurations of a syringe tip **2:** a luer lock tip **8** and a slip tip **9.** The luer lock tip **8** provides a male fitting with threading such that a female needle hub is twisted onto the luer lock tip. The slip tip **9** provides a male fitting configured such that a female needle hub is slipped over and fitted onto the slip tip. In an embodiment, a syringe comprises a plastic syringe barrel, a plunger rod, a plunger stopper, and a needle. In an embodiment, the needle is staked needle or pre-attached to the syringe barrel. In an embodiment, the needle is glued to the syringe barrel. In an embodiment, a syringe comprises a plastic syringe barrel, a luer lock tip or a slip tip, a plunger rod, and a plunger stopper.

FIG. 1A-2 is an image of an exemplary syringe comprising a plastic syringe barrel **1,** a syringe tip **2,** a plunger rod **3,** a plunger stopper **4,** and a staked (or pre-attached) needle **6a,** a needle safety cap **7,** and a plunger back cap **15** (left); and an image of the same with the needle safety cap **7** and plunger back cap **15** assembled or capped on (right).

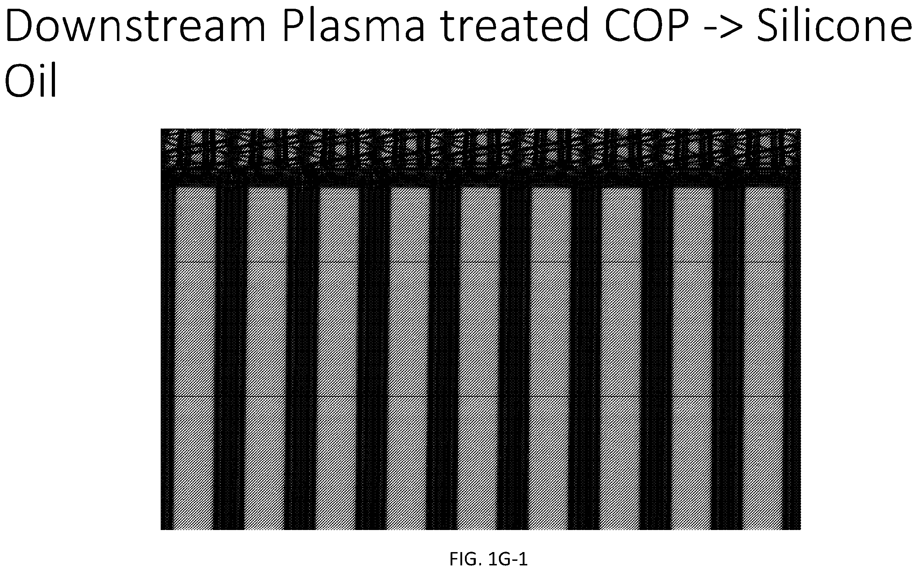

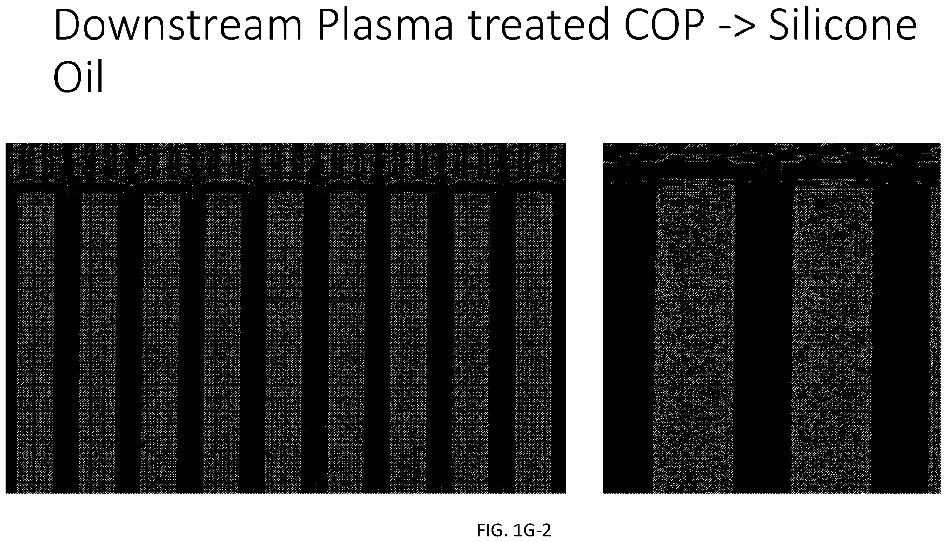

FIG. 1B is cross-section view of a plastic syringe barrel **1** and a syringe tip **15** (left). The plastic syringe barrel **1** comprises a proximal end **10,** a distal end **11,** a cylindrical wall **12** extending between the proximal end and distal end. The cylindrical wall **12** of the syringe barrel has an interior surface **13** and defines a chamber **14** for receiving a substance (e.g. a solution). According to embodiments of the invention, the interior surface **13** is an interior surface of a plasma-treated plastic syringe barrel with plasma-treated silicon oil coating, or an interior surface of a plastic syringe barrel treated with plasma and coated with a polysiloxane-based lubricant coating treated with plasma, wherein each plasma treatment consisted essentially of uncharged energized gaseous species. See for example FIG. 1G and FIG. 1H. The cross-section diagrams on the right of FIG. 1B illustrate the various surfaces: the interior surface of a plastic syringe barrel **17** to be plasma treated and the interior space of a plastic syringe barrel **18,** a plasma-treated plastic syringe barrel **19,** a uniform polysiloxane-based lubricant coating **20** (e.g. a uniform silicone oil coating) to be plasma treated, the interior surface **21** of the plasma-treated plastic syringe barrel with plasma-treated polysiloxane-based lubricant coating (e.g. plasma-treated plastic syringe barrel with plasma-treated silicone oil coating) from which surface density particle images and measurements are taken, and the plasma-treated polysiloxane-based lubricant coating **22.** The syringe barrel chamber **14** may be pre-filled with a medicament in either dry or liquid form, an ophthalmic solution, a biologic, or any other substances including water or diluent used in reconstituting a medicament. The distal end **11** of the syringe barrel is connected to a syringe tip **15** having a passage **16** extending therethrough and communicating with the syringe barrel chamber **14.** A plunger rod **3** (shown in FIG. 1A) may extend into the proximal end **10** of the plastic syringe barrel **1,** wherein the plunger stopper **4** slides in fluid-tight engagement inside the cylindrical wall **12** of the chamber **14.**

Described herein are exemplary embodiments of a method of producing a plastic syringe barrel with a stable polysiloxane-based lubricant layer comprising providing a plastic syringe barrel, exposing an interior surface of the plastic syringe barrel to a plasma consisting essentially of uncharged energized gaseous species; applying a polysiloxane-based lubricant coating to the plasma-treated interior surface of the plastic syringe barrel to form a uniform polysiloxane-based lubricant coating; and exposing the uniform polysiloxane-based lubricant coating to a plasma consisting essentially of uncharged energized gaseous species. The method surprisingly results in extremely low surface density particle counts on the interior surface of the plasma-treated plastic syringe barrel with the plasma-treated polysiloxane-based lubricant coating, wherein the plastic syringe barrel is unfilled or empty. In an embodiment, the interior surface of the plasma-treated plastic syringe barrel with the plasma-treated polysiloxane-based lubricant coating has at least a 95% reduction in the number of particles greater than 8 microns in diameter as compared to an interior surface of a plastic syringe barrel with a polysiloxane-based lubricant coating. In an embodiment, the interior surface of the plasma-treated plastic syringe barrel with plasma-treated polysiloxane-based lubricant has a surface density of ≤ 600 particles per 12 cm², wherein the particles are greater than 8 microns in diameter. ("12 cm²" as used herein and throughout is the approximate total surface area of the interior surface of a 1ml plastic syringe barrel, and the term "12 cm²" generally encompasses the total surface areas of the interior surface of various configurations 1ml plastic syringe barrels). In an embodiment, the interior surface of the plasma-treated plastic syringe barrel with the plasma-treated polysiloxane-based lubricant coating has a surface density of ≤ 30 particles per cm², wherein the particles are greater than 8 microns in diameter.

While it is known that plasma treatment of plastic can enhance wettability of fluids that have reactive functional groups or have polarity, it is an uncommon in industry practice to treat a plastic surface with plasma because the extra processing step is inconvenient without any known significant upside or superior lubricant stability. Importantly, one skilled in the art would not expect the plasma treatment of plastic to enhance wettability by the commonly used silicone oil lubricant which is non-polar and does not possess any reactive functional groups. Plastic materials such as polypropylene and other cyclic olefin polymers commonly used for manufacturing of syringes are difficult to bond because they are hydrophobic, possesses poor surface wettability (low surface energies), do not have any surface reactive functional groups and are non-polar. This non-reactive or inert property of these plastics is important to maintain stability of the drug product which are in direct contact with the plastic materials. Downstream plasma treatment of plastic improves wettability by slightly raising the plastic's surface energy by creating functional groups on the surface; *however,* because silicone oil is non-polar without any functional groups, one skilled in the art would expect a slight change in plastic wettability to only provide incremental improvements in silicone oil wettability but no further improvement in chemical bonding between the plastic and silicone oil. Since there is no bonding between the silicone oil and the plastic surface, the lubricant can easily migrate under mechanical or chemical stress. Mechanical stress means the movement of the plunger rod in the syringe barrel and chemical stress is the contact of polar fluids such as aqueous based drug products. Due to the huge difference in surface energy between aqueous solutions and the non-polar and inert silicone oil, the lubricant tends to recede into micro-droplets on the plastic surface to reduce the surface area of contact between the silicone oil and the drug fluid. Since there are no permanent chemical bonds between the silicone oil and the plastic, the silicone oil can easily migrate on the plastic surface or from the plastic surface into the drug solution. Consistent with these expectations, product development of plastic syringes has moved away from use of silicone oil or plasma treatment. This is evident by industry product development generally focused on silicone-free plastic syringes (e.g. West Daikyo Crystal Zenith (CZ^{®}), and plastic syringes utilizing chemically crosslinked reactive silicone oil instead (e.g. Schott TopPac^{®}).

The disclosures herein demonstrate the unexpected result that the combination of plasma treatment of plastic as described herein first increases the wettability of the silicone oil such that the silicone layer flattens out to a film rather than stay as discrete droplets or islands on the surface, followed by the subsequent plasma treatment of the lubricant layer as described herein which results in the permanent cross-linking of the lubricant film to produce a permanent coating as opposed to cross-linked particles. The plasma induced crosslinking of the lubricant arrests the further mobility of the silicone oil that remains as a uniform coating even under mechanical or chemical stresses as described above. This in turn provides the unexpected result of silicone oil lubricated plastic syringe barrels having extremely low particle counts on the interior surface, and filled silicone oil lubricated plastic syringe barrels having extremely low particle counts in solution.

As used herein, "a plasma consisting essentially of uncharged energized gaseous species" refers to a plasma limited to the recited uncharged energized gaseous species and possibly charged energized gaseous species that do not materially affect the basic and novel characteristics of the plasma consisting essentially of uncharged energized gaseous species. These basic and novel characteristic is the ability to produce at least one of the following, as applicable or recited in the respective claim: a polysiloxane-lubricated plastic syringe barrel with an interior surface with at least a 95% reduction in the number of particles greater than 8 microns in diameter as compared to an interior surface of a plastic syringe barrel with a polysiloxane-based lubricant coating; a polysiloxane-lubricated plastic syringe barrel with an interior surface having a surface density of ≤ 600 particles per 12 cm² for particles greater than 8 microns in diameter; a polysiloxane-lubricated plastic syringe barrel with an interior surface having a surface density of ≤ 50 particles per cm², wherein the particles are greater than 8 microns in diameter; or a polysiloxane-lubricated plastic syringe barrel that can contain a solution with a particle level that is ≤ 50 particles per ml for any particles of a diameter of ≥ 10µm or ≤ 5 particles per ml for any particles of a diameter of ≥ 25µm.

In an embodiment, the uncharged energized gaseous species comprises excited argon gas atoms. In an embodiment, the reactive gas is oxygen. In another embodiment the reactive gas is air. In another embodiment mixtures of reactive gasses with argon is used. In an embodiment, the method comprises exposing the interior surface of the plastic syringe barrel to a plasma consisting essentially of uncharged energized gaseous species for 0.1 seconds to 10 seconds; and applying 0.005mg/cm² to 0.5mg/cm² of silicone oil to the plasma-treated interior surface of the plastic syringe barrel to form a uniform silicone oil coating.

In an embodiment, the plastic syringe barrel is comprised of cyclic olefin polymer (COP), cyclic olefin copolymer (COC), polyethylene (PE), polycarbonate (PC), polypropylene (PP), or polyethylene terephthalate (PET). In an embodiment, the plastic syringe barrel has a maximum fill volume of 10ml, 3ml, 1ml, 0.5ml, 0.3ml, 0.25ml, 0.10ml, or 0.05ml.

The method of producing a plastic syringe barrel with a stable silicone oil layer comprises exposing the plastic syringe barrel interior surface to a plasma consisting essentially of uncharged energized gaseous species. In an embodiment, the plasma consisting essentially of uncharged energized gaseous species is a downstream plasma produced for example, by the configuration for a plasma process described in U.S. Patent No. 9133412. The downstream plasma configuration described in U.S. Patent No. 9133412 generates a gas stream with a mixture of uncharged energized gaseous species and charged species in one location, then flows filtered or separated gas plasma enriched with uncharged energized gaseous species "downstream" to a second location where an article is plasma-treated. U.S. Patent No. 9133412 describes treating a lubricant with downstream plasma, and further describes that thermal and electronic energy may be released locally by the energized gaseous species creating reaction sites among the lubricant molecules, nearly instantaneously or through a continued reaction process, which may produce desirable material properties.

A downstream plasma consisting essentially of uncharged energized gaseous species is distinct from the atmospheric plasma process described in U.S. Patent No. 7553529 and the vacuum plasma processes described in U.S. Patent Application No. 14/347677 and U.S. US4767414, all of which describe a direct ionizing plasma radiation process that may cause retained embedded charges at or near the treated surface and alter material properties of the treated surface differently.

In an embodiment, the uncharged energized gaseous species comprise highly energetic neutrals, free radicals, neutral atoms or molecules formed from electrons and ions combining, or excited noble gas atoms. In an embodiment, the free radicals are reactive gas atoms or polymerizable gas atoms.

In an embodiment, the downstream plasma or plasma consisting essentially of uncharged energized gaseous species is produced in part by an initial gas stream comprising a noble gas. In an embodiment, the noble gas is helium, neon, argon, or krypton. In an embodiment, the downstream plasma or plasma consisting essentially of uncharged energized gaseous species is produced in part by an initial gas stream comprising an oxidative gas. In an embodiment, the oxidative gas is air, oxygen, carbon dioxide, carbon monoxide, water vapor, or mixtures thereof. In an embodiment, the downstream plasma or plasma consisting essentially of uncharged energized gaseous species is produced in part by an initial gas stream comprising a non-oxidative gas. In an embodiment, the non-oxidative gas is nitrogen or hydrogen. In an embodiment, the downstream plasma or plasma consisting essentially of uncharged energized gaseous species is produced in part by an initial gas stream comprising a mixture of gases.

In an embodiment, the downstream plasma or plasma consisting essentially of uncharged energized gaseous species is produced in part by a gas plasma and a mixture of charged and uncharged energized gaseous species generated by microwave energy, a high-voltage direct current (DC), radio frequency (RF) power supply, or a thermal activation process, such as passing a gas over a catalytic surface or a heated wire. Examples of devices that generate energized gaseous species include a capacitively coupled plasma generating device with two counter electrodes, an inductively coupled plasma generating device having a coil encircling a gas stream, a microwave generator electrically coupled to a power supply to produce electromagnetic radiation that energizes a gas stream, a catalyzer comprising a wire or other electrically resistive material coupled to a power supply.

In an embodiment, the downstream plasma or plasma consisting essentially of uncharged energized gaseous species is produced in part by separating or filtering uncharged energized gaseous species from ions, electrons, and other charged species by electrical grounding (e.g. with one or more wires, a screen, a mesh, or any other structure known in the art that is electrically conductive and may be electrically grounded), by one or more electrostatic or electromagnetic fields, or by neutralizing charged species in the gas stream via recombination in a transfer zone.

In an embodiment, the downstream plasma or plasma consisting essentially of uncharged energized gaseous species is generated under vacuum (generally less than about 200 torr) or at about atmospheric pressure (generally about 760 torr).

The method of producing a plastic syringe barrel with a stable polysiloxane-based lubricant further comprises applying a polysiloxane-based lubricant to the plasma-treated interior surface of the plastic syringe barrel to form a uniform polysiloxane-based lubricant coating. In an embodiment, the method comprises applying 0.005mg/cm²-0.05mg/cm² of a polysiloxane-based lubricant to the plasma-treated interior surface of the plastic syringe barrel. In an embodiment, the polysiloxane-based compound is a silicone oil with a dimethlypolysiloxane chemical formulation of the following general chemical structure: 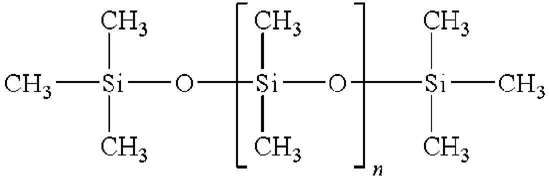 where n = 10-50,000
The number of repeating siloxane units (n) in the polymer chain will determine the molecular weight and viscosity of the silicone oil. As the number of siloxane units increases, the polymer becomes longer and both the molecular weight and viscosity increases. Generally, the usable viscosity range of silicone oils is about 5-100,000 centistokes at ambient temperature. Preferably, the viscosity of the polysiloxane-based lubricant is about 1000-12500 centistokes at ambient temperature. Preferably, the polysiloxane-based lubricant is polydimethylsiloxane (PDMS), 1000 centistokes at ambient temperature. In another embodiment the method comprises of using other non-silicone inert lubricants. These include inert fluorochemical lubricants such as perfluoropolyether (PFPE). Representative examples of commercially available PFPE include Fomblin M^{®}, Fomblin Z^{®}, Fomblin Y^{®} families of lubricant from Solvay Solexis; Krytox^{®} family of lubricants from E.I. du Pont de Nemours and Company; and Demnum^{®} from Daikin Industries, Ltd. Uniform coatings may be achieved by heating the lubricant immediately prior to application, adding solvent, or mechanically wiping the lubricant after spraying.

The lubricant can be applied in a diluted or non-diluted form, and combinations of diluted or non-diluted lubricants can be used. In an embodiment, the silicone oil lubricant is applied as a water dispersion or as an emulsion. Any suitable solvent can be used as the diluent that is compatible with the lubricant or combination of lubricants used. The lubricant may be diluted in order to facilitate the application of a thin film of the lubricant onto the surface of the object. The amount of dilution, or weight percent of lubricant in the lubricant-solvent solution, is not essential to the performance of the invention. The weight percent of lubricant in the solvent, when a solvent is used, may be greater than or equal to about 0.1 percent, such as, for example, 1, 10, 20, 30, 40 and 50. The weight percent of the lubricant in the solvent may also be less than or equal to about 95 percent, such as, for example, 90, 80, 70, and 60. The diluent solvent is evaporated prior to exposure to the downstream plasma or plasma consisting essentially of uncharged energized gaseous species.

The method of producing a plastic syringe barrel with a stable polysiloxane-based lubricant layer further comprises exposing the uniform silicone oil coating to a downstream plasma or downstream plasma consisting essentially of uncharged energized gaseous species. The term "plasma consisting essentially of uncharged energized gaseous species" is described above. In an embodiment, the resulting interior surface of the plasma-treated plastic syringe barrel with plasma-treated polysiloxane-based lubricant has a surface density of ≤ 600 particles per 12 cm² for any particles greater than 8 microns in diameter. In an embodiment, the resulting interior surface of the plasma-treated plastic syringe barrel with plasma-treated polysiloxane-based lubricant has a surface density of ≤ 40 particles per cm² for particles greater than 8 microns in diameter. In an embodiment, the resulting interior surface of the plasma-treated plastic syringe barrel with plasma-treated polysiloxane-based lubricant has at least a 95% reduction in particles as compared to an interior surface of a plastic syringe barrel with a silicone oil.

In another embodiment, the method of producing a plastic syringe barrel with a stable polysiloxane-based lubricant layer further comprises assembling the plastic syringe barrel with a stable polysiloxane-based lubricant layer with a plunger rod, plunger stopper, and needle. Due to the surprising and extremely low particle counts on the interior surface of the syringe as well as in solution, the plastic syringes according to embodiments of the invention are particularly advantageous for user-filled syringes (which require more plunger rod movements to aspirate and deliver a drug) and pre-filled ophthalmic syringes (which are regulated to ensure extremely low subvisible particle levels).

In an embodiment, the syringe contains a solution comprising a biologic. In an embodiment, the syringe contains an ophthalmic solution. In an embodiment, the syringe contains an ophthalmic solution or a solution comprising a biologic, and the particle level in the solution is ≤ 50 particles per ml for any particles of a diameter of ≥ 10µm or ≤ 5 particles per ml for any particles of a diameter of ≥ 25µm.

Described herein are exemplary embodiments of a plastic syringe comprising a plastic barrel, a plunger rod, a plunger stopper, and needle wherein the plastic barrel has a stable polysiloxane-based lubricant layer and is produced by: exposing an interior surface of the plastic syringe barrel to a plasma consisting essentially of uncharged energized gaseous species for 0.1second to 10 seconds, applying 0.005mg/cm²-0.5mg/cm² of a polysiloxane-based lubricant to the plasma-treated interior surface of the plastic syringe barrel to form a uniform polysiloxane-based lubricant coating; and exposing the uniform polysiloxane-based coating to a plasma consisting essentially of uncharged energized gaseous species. Advantageously, the interior surface of the plasma-treated plastic syringe barrel with plasma-treated polysiloxane-based lubricant has a surface density of ≤ 600 particles per 12 cm² for particles greater than 8 microns in diameter. Advantageously, the interior surface of the plasma-treated plastic syringe barrel with plasma-treated polysiloxane-based lubricant has a surface density of ≤ 40 particles per cm² for particles greater than 8 microns in diameter. Advantageously, the interior surface of the plasma-treated plastic syringe barrel with the plasma-treated silicone oil has at least a 95% reduction in particles as compared to an interior surface of a plastic syringe barrel with a silicone oil.

Described herein are exemplary embodiments of a plastic syringe barrel comprising a plastic syringe barrel treated with plasma and coated with a polysiloxane-based lubricant coating treated with plasma, wherein each plasma is a downstream plasma generated at atmospheric pressure; and wherein the interior surface of the plasma-treated plastic syringe barrel with the plasma-treated polysiloxane-based lubricant coating has a surface density of ≤ 600 particles per 12 cm², ≤ 500 particles per 12 cm², or ≤ 400 particles per 12 cm², wherein the particles are greater than 8 microns in diameter..

Further described herein are exemplary embodiments of a syringe comprising a plastic syringe barrel comprising a plastic syringe barrel treated with plasma and coated with a polysiloxane-based lubricant coating treated with plasma, wherein each plasma treatment consisted essentially of uncharged energized gaseous species; and wherein the interior surface of the plasma-treated plastic syringe barrel with the plasma-treated polysiloxane-based lubricant coating has a surface density of ≤ 100 particles per cm², ≤ 90 particles per cm², ≤ 50 particles per cm², ≤ 40 particles per cm², ≤ 35 particles per cm², or ≤ 30 particles per cm² wherein the particles are greater than 8 microns in diameter; the syringe further comprising a plunger rod, a plunger stopper, and a needle.

Further described herein are exemplary embodiments of a syringe comprising a plastic syringe barrel comprising a plastic syringe barrel treated with plasma and coated with a polysiloxane-based lubricant coating treated with plasma, wherein each plasma treatment consisted essentially of uncharged energized gaseous species; and wherein the interior surface of the plasma-treated plastic syringe barrel with the plasma-treated polysiloxane-based lubricant coating has a surface density of ≤ 100 particles per cm², ≤ 90 particles per cm², ≤ 50 particles per cm², ≤ 40 particles per cm², ≤ 35 particles per cm², or ≤ 30 particles per cm² wherein the particles are greater than 8 microns in diameter; the syringe further comprising a luer lock tip or slip tip, a plunger rod, and a plunger stopper. In an embodiment, the plastic syringe barrel of size 0.25ml capacity comprises about 50micrograms to about 500micrograms of silicone oil. In an embodiment, the plastic barrel contains a solution and the particle level in the solution is ≤ 50 particles per ml for any particles of a diameter of ≥ 10µm or ≤ 5 particles per ml for any particles of a diameter of ≥ 25µm. In an embodiment, the plastic barrel contains a solution comprising a biologic. In an embodiment, the biologic is an anticoagulant, vaccine, or recombinant protein. In an embodiment, the biologic is pegaptanib, ranibizumab, aflibercept, or bevacizumab. In an embodiment, the plastic barrel contains an ophthalmic solution and the particle level in the ophthalmic solution is ≤ 50 particles per ml for any particles of a diameter of ≥ 10µm or ≤ 5 particles per ml for any particles of a diameter of ≥ 25µm. In an embodiment, the plastic syringe barrel has a maximum fill volume of 1ml, 0.5ml, 0.3ml, 0.25ml, 0.10ml, or 0.05ml. Further described herein is a method treating the eye, comprising intravitreally administering the ophthalmic solution to an eye with a plastic syringe according to embodiments described herein.

### EXAMPLE 1: Extremely low particle counts on the interior surface of a plastic syringe barrel

### Materials/Methods:

A COP syringe barrel, a downstream plasma-treated COP syringe barrel with sprayed-on silicone oil, and a downstream plasma-treated COP syringe barrel with downstream plasma-treated silicone oil were prepared.

### Step 1: Plasma treatment of COP syringe barrel:

1ml COP syringe barrels were treated with a down-stream plasma.
**a.** Syringe format - 1ml luer lock
**b.** Gas used - Argon
**c.** Gas flow rate - 3 standard liters per min flowing continuously through the syringe barrel. Pressure inside syringe barrel was at about atmospheric pressure as there was no tip cap installed on the syringe during downstream plasma treatment. Gas flow direction was from flange end with gas purging out of the luer end. Syringe was allowed to purge with Argon for 2 seconds.
**d.** Downstream Plasma was initiated for 1.5 seconds.

### Step 2: Application of silicone oil to two of the plasma-treated COP syringe barrels:

1000cSt of Dow Corning DC360 medical fluid was sprayed onto the interior surface of the COP syringe barrels using IVEK Sonicair spray instrument. Spray nozzle was heated to 150 degrees F (~ 66 degrees C). A total of 0.4 microliters of DC360 oil was sprayed into the 1ml COP syringe at the rate of 0.4 microliters/sec. Total spray duration was 1 sec. During the spray step the syringe barrel was concurrently moved in the vertical direction such that the nozzle entered into the syringe barrel to result in a uniform spray pattern along the internal surface of the syringe barrel. The start and stop positions of the syringe barrel during spray were adjusted to result in a uniform spray coverage.

### Step 3: Plasma treatment of COP syringe barrels with silicone oil:

**a.** Gas used - Argon
**b.** Gas flow rate - 3 standard liters per min flowing continuously through the syringe barrel. Pressure inside syringe barrel was at about atmospheric pressure as there was no tip cap installed on the syringe during downstream plasma treatment. Gas flow direction was from flange end with gas purging our of the luer end. Syringe was allowed to purge with Argon for 2 seconds.
**c.** Downstream Plasma was initiated for 0.5 seconds.

### Zebrasci Flex-S Imaging:

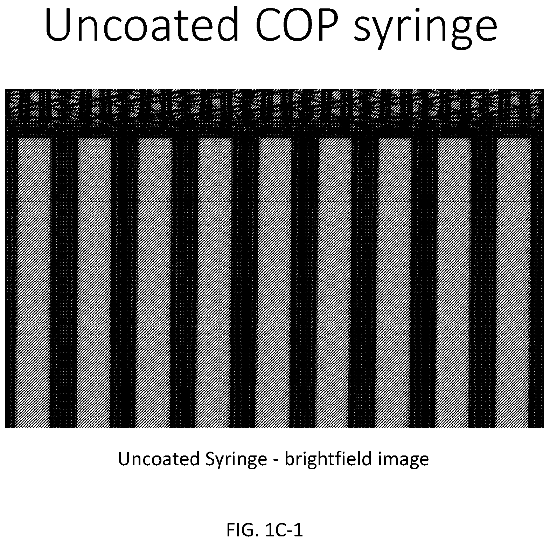

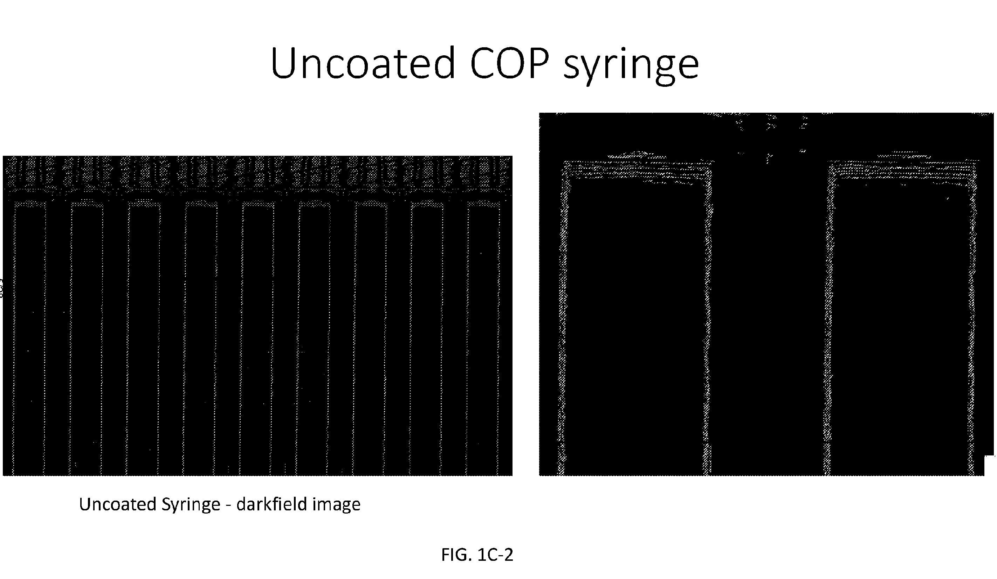

The interior surface of each of the sample COP syringe barrels were imaged using the camera-based inspection tool ZebraSci Flex S Bench-Top Combination Spray System, methods, and algorithms. The imaging system imaged each syringe barrel and took multiple high resolution images of the syringes (note that with respect to the plasma-treated COP syringe barrel with sprayed-on silicone oil, the images were immediately taken after application of the silicone oil). The imaging system utilizes a backlight paired with a light mask and a camera, and the mask produces a light pattern with alternating dark and light regions to enable detection of changes in the refractive index. Brightfield images were produced wherein light was reflected into the camera, and darkfield images were produced wherein light was reflected away from the camera. The imaging system identified edge definitions of silicone oil droplets or particles on the surface through multiple images that are stitched together to display the entire mapped surface of the syringe. In each of FIGS. 1C-H, and 1J, the multiple vertical rows represent images of the same syringe barrel, and each row is a stitched or compiled image of multiple images (roughly 18 images) taken at various rotations of the syringe barrel (6 vertically, 6 horizontally, 6 in z direction).

**Example 1a**: Samples: empty/unfilled uncoated COP syringe barrel (no steps), empty/unfilled COP syringe barrel with sprayed-on silicone oil (COP syringe barrel treated with Step 2), and empty/unfilled downstream plasma-treated COP syringe barrel with downstream plasma-treated silicone oil (COP syringe barrel treated with Steps 1-3). With respect to the COP syringe barrel with sprayed-on silicone oil, Zebrasci images were taken 0-10 minutes after spraying. With respect to the downstream plasma-treated COP syringe barrel with downstream plasma-treated silicone oil - between 30min to 1hr lapsed between steps 2 and 3. The interior surface of the syringe barrel was imaged with ZebraSci Flex S Bench-Top Combination Spray System, methods, and algorithms.

**Example 1b**: Samples: empty/unfilled downstream plasma-treated COP syringe barrel with sprayed-on silicone oil (COP syringe barrel treated with Steps 1 and 2); empty/unfilled COP syringe barrel with downstream plasma-treated silicone oil (COP syringe barrel treated with Steps 2 and 3); empty/unfilled downstream plasma-treated COP syringe barrel with downstream plasma-treated silicone oil (COP syringe barrel treated with Steps 1-3). With respect to the downstream plasma-treated COP syringe barrel with downstream plasma-treated silicone oil - between 0-10min lapsed between steps 2 and 3. The interior surface of the syringe barrel was imaged with ZebraSci Flex S Bench-Top Combination Spray System, methods, and algorithms.

**Prophetic Example 1c:** Samples: multiple downstream plasma-treated COP syringe barrels with downstream plasma-treated silicone oil (COP syringe barrel treated with Steps 1-3). Processing times will be tested, including the following:

| **Syringe barrel #** | **Time lapse between Step 1 and Step 2** | **Time lapse between Step 2 and Step 3** |
|---|---|---|
| 1 | 0-10min | 0-10min |
| 2 | 0-10min | 1hr |
| 3 | 0-10min | 2hrs |
| 4 | 0-10min | 3hrs |

The interior surface of the syringe barrels will be imaged with ZebraSci Flex S Bench-Top Combination Spray System, methods, and algorithms.

Particle counts in solution will be measured: Syringe will be filled with water for injection (WFI) Particles in solution will be measured by light obscuration using a Accusizer 780 device and micro-flow imaging using a MFI 5200 device. Measurements will be performed to comply with standard USP protocols for particulate measurements or per instrument manufacturer's recommended settings. Particle testing will include filling syringes to 0.05ml through the syringe needle, dispensing the solution into a cleaned tube at time 0, diluting contents to 5ml with WFI, allowing the tube to stand for about 1hr to reduce bubbles, and vortex mixing the tube just prior to measurement. Particle analysis by MFI will include analyzing images to identify types of particles. Silicone oil micro-droplets have an aspect ratio of ≥ 0.85; and other particles have an aspect ratio <85. The unique processing times will further improve and further lower lower particle counts in solution as the short time lapse between Step 1 and Step 2 enables use of the surface functional groups created, and the longer time lapse between Step 2 and Step 3 enables the silicone oil to flatten out on the surface before the final plasma treatment.

### Example 1d: Surface density particle count calculations:

Samples: uncoated COP syringe barrel (no steps), COP syringe barrel with sprayed-on silicone oil (COP syringe barrel treated with Step 2), and downstream plasma-treated COP syringe barrel with downstream plasma-treated silicone oil (COP syringe barrel treated with Steps 1-3).

Images of the interior surface of these syringe barrel samples were obtained with the Flex S Bench-Top Combination Spray System, methods, and algorithms, and then the images were further characterized by the system to count the number of particles and provide surface density measurements of particles per cm². The resolution of the system for particle counting is 7.33 microns per pixel; thus, the smallest particle size measured would be 7.33 microns.

### Example 1a Results:

Images of the interior surface of an empty/unfilled uncoated COP syringe barrel, the interior surface of an empty/unfilled COP syringe barrel with sprayed-on silicone oil, and the interior surface of an empty/unfilled downstream plasma-treated COP syringe barrel with downstream plasma-treated silicone oil are shown in the figures as described below. Features which are due to silicone oil droplets (white) are more easily visualized in the darkfield images. These droplets present on the surface are referred to as particles on the surface.

FIG. 1C-1 are brightfield images of the interior surface of an empty/unfilled COP syringe barrel without any coating.

FIG. 1C-2 are corresponding darkfield images of the interior surface of the empty/unfilled COP syringe barrel without any coating (left), and an enlarged view of a portion of those darkfield images (right).

FIG. 1D-1 are brightfield images of the interior surface of an empty/unfilled COP syringe barrel after spray of 1000cSt silicone oil.

FIG. 1D-2 are corresponding darkfield images of the interior surface of the empty/unfilled COP syringe barrel with sprayed on silicone oil (left), and an enlarged view of a portion of those darkfield images (right). The micro-droplets of silicone oil are shown in abundance which are seen as droplet features on the syringe internal surface.

FIG. 1E-1 are brightfield images of the interior surface of an empty/unfilled plasma-treated COP syringe barrel with plasma-treated silicone oil according to an embodiment of the invention. The plasma treatment of the COP syringe barrel and the plasma treatment of the silicone oil were each a plasma treatment consisting essentially of uncharged energized gaseous species.

FIG. 1E-2 are corresponding darkfield images of the interior surface of the empty/unfilled plasma-treated COP syringe barrel with plasma-treated silicone oil according to an embodiment of the invention (left), and an enlarged view of a portion of those darkfield images (right). The plasma treatment of the COP syringe barrel and the plasma treatment of the silicone oil were each a plasma treatment consisting essentially of uncharged energized gaseous species.

FIG. 1F-1 are enlarged brightfield images of the interior surface of the empty/unfilled COP syringe barrel without any coating (left), the interior surface of the empty/unfilled COP syringe barrel with sprayed on silicone oil (middle), the interior surface of the empty/unfilled plasma-treated COP syringe with plasma-treated silicone oil according to an embodiment of the invention, wherein the plasma treatment of the COP syringe barrel and the plasma treatment of the silicone oil were each a plasma treatment consisting essentially of uncharged energized gaseous species (right).

FIG. 1F-2 are enlarged darkfield images of the interior surface of the empty/unfilled COP syringe barrel without any coating (left), the interior surface of the empty/unfilled COP syringe barrel with sprayed on silicone oil (middle), and the interior surface of the empty/unfilled plasma-treated COP syringe with plasma-treated silicone oil according to an embodiment of the invention, wherein the plasma treatment of the COP syringe barrel and the plasma treatment of the silicone oil were each a plasma treatment consisting essentially of uncharged energized gaseous species (right). As shown, the interior surface of the empty/unfilled plasma-treated COP syringe with plasma-treated silicone oil is surprisingly comparable to the interior surface of the empty/unfilled plasma-treated COP syringe barrel without any coating, which has no silicone oil droplets. As shown, the interior surface of the empty/unfilled plasma-treated COP syringe barrel with the plasma-treated silicone oil surprisingly has at least a 95% reduction in particles as compared to the interior surface of the empty/unfilled COP syringe barrel with sprayed on silicone oil.

### Example 1b results:

Images of the interior surface of the empty/unfilled downstream plasma-treated COP syringe barrel with sprayed-on silicone oil (COP syringe barrel treated with Steps 1 and 2); the interior surface of the empty/unfilled COP syringe barrel with downstream plasma-treated silicone oil (COP syringe barrel treated with Steps 2 and 3); and the interior surface of the empty/unfilled downstream plasma-treated COP syringe barrel with downstream plasma-treated silicone oil (COP syringe barrel treated with Steps 1-3) are shown in the figures as described below. Features which are due to silicone oil droplets (white) are more easily visualized in the darkfield images. These droplets present on the surface are referred to as particles on the surface.

FIG. G-1 are brightfield images of the interior surface of an empty/unfilled downstream plasma-treated COP syringe barrel with sprayed-on silicone oil.

FIG. G-2 are corresponding darkfield images of the interior surface of the empty/unfilled downstream plasma-treated COP syringe barrel with sprayed-on silicone oil (left), and an enlarged view of a portion of those darkfield images (right)

FIG. 1H-1 are brightfield images of the interior surface of an empty/unfilled COP syringe barrel with downstream plasma-treated silicone oil.

FIG. 1H-2 are corresponding darkfield images of the interior surface of the empty/unfilled COP syringe barrel with downstream plasma-treated silicone oil (left), and an enlarged view of a portion of those darkfield images (right)

FIG. 1I are darkfield images of the interior surface of the empty/unfilled COP syringe barrel without any coating (left), the interior surface of the empty/unfilled downstream plasma-treated COP syringe barrel with sprayed-on silicone oil (middle), and the interior surface of the empty/unfilled plasma-treated COP syringe with plasma-treated silicone oil according to an embodiment of the invention, wherein the plasma treatment of the COP syringe barrel and the plasma treatment of the silicone oil were each a plasma treatment consisting essentially of uncharged energized gaseous species (right)

FIG. 1J are darkfield images of the interior surface of the empty/unfilled COP syringe barrel without any coating (left), the interior surface of the empty/unfilled COP syringe barrel with downstream plasma-treated silicone oil (middle), and the interior surface of the empty/unfilled plasma-treated COP syringe with plasma-treated silicone oil according to an embodiment of the invention, wherein the plasma treatment of the COP syringe barrel and the plasma treatment of the silicone oil were each a plasma treatment consisting essentially of uncharged energized gaseous species (right).

### Example 1d results:

Surface density particle counts were obtained for the interior surface of an empty/unfilled uncoated COP syringe barrel (no steps), the interior surface of an empty/unfilled COP syringe barrel with sprayed-on silicone oil (COP syringe barrel treated with Step 2), and the interior surface of an empty/unfilled downstream plasma-treated COP syringe barrel with downstream plasma-treated silicone oil (COP syringe barrel treated with Steps 1-3).

FIG. 1K are darkfield images (left) and brightfield images (right) of the interior surface of an empty COP syringe barrel without any coating. The corresponding surface density particle count was 3 particles/cm sq. on the interior surface of the empty uncoated COP syringe barrel.

FIG. 1L are darkfield images (left) and brightfield images (right) of the interior surface of an empty COP syringe barrel immediately after spray of 1000cSt silicone oil. The corresponding surface density particle count was 31,419 particles/cm sq on the interior surface of the empty COP syringe barrel with sprayed-on silicone oil.

FIG. 1M are brightfield images (left), darkfield images (middle) of the interior surface of an empty plasma-treated COP syringe barrel with plasma-treated silicone oil according to an embodiment of the invention (middle), and an enlarged view of a portion of those darkfield images (right). The plasma treatment of the COP syringe barrel and the plasma treatment of the silicone oil were each a plasma treatment consisting essentially of uncharged energized gaseous species. The corresponding surface density particle count was 30 particles/cm sq. on the interior surface of empty downstream plasma-treated COP syringe barrel with downstream plasma-treated silicone oil.

### EXAMPLE 2 - Particle count with WFI

Testing was performed on 0.25 mL polypropylene syringes with an attached 31G needle. The syringe types tested were:
- Becton Dickenson U100 insulin syringes, and
- General use 0.25ml StaClear syringes as an embodiment of this invention and processed as described below

The 0.25ml polypropylene syringes were processed according to the following steps:
- Plastic barrel (without any silicone oil lubricant) was treated with a downstream plasma consisting essentially of uncharged energized gaseous species. Argon gas was purged into the syringe barrel at a rate of 3 standard liters per min. After a purge time of at least 1 second of gas flow, the downstream plasma was energized for 1.5seconds of treatment time.
- Dow Corning DC360 silicone oil of viscosity 1000cSt was applied to the downstream plasma-treated interior surface of the plastic syringe barrel by using the IVEK Sonicair spray equipment. Spray nozzle was heated to 150 degrees F (~66 degrees C) and a total lubricant volume of 0.15 microliters was sprayed uniformly on the inside of the syringe barrel at a rate of 0.15 microliters/sec. Syringe was moved in the vertical direction concurrently with the spray trigger such that the spray nozzle inserted into the syringe barrel to give uniform coverage of the spray.
- The lubricated interior surface of the plastic syringe was then treated with a downstream plasma consisting essentially of uncharged energized gaseous species. Argon gas was purged into the syringe barrel at a rate of 3 standard liters per min. After a purge time of at least 1 second of gas flow, the downstream plasma was energized for 0.5seconds of treatment time.

10 syringes of each type of syringe were filled with water for injection (WFI) Particles in solution were measured by light obscuration using a Accusizer 780device and micro-flow imaging using a MFI 5200 device. Measurements were performed to comply with standard USP protocols for particulate measurements or per instrument manufacturer's recommended settings. Particle testing included filling syringes to 0.05ml through the syringe needle, dispensing the solution into a cleaned tube [at time 0], diluting contents to 5ml with WFI, allowing the tube to stand for about 1hr to reduce bubbles, and vortex mixing the tube just prior to measurement. Particle analysis by MFI included analyzing images to identify types of particles. Silicone oil micro-droplets have an aspect ratio of ≥ 0.85; and other particles have an aspect ratio <85.

Table 1 below reports the Light Obscuration measurements of cumulative particle concentration (number of particles per mL) of particles ≥ 2µm, particles ≥ 5µm, particles ≥ 10µm, particles ≥ 25µm, particles ≥ 50µm between the following solution sources: WFI stock solution in a clean container, BD U100 insulin syringes filled with WFI, and general use (StaClear) 0.25ml syringe treated according to the method above and filled with WFI.

**Table 1: Light Obscuration**

| **Size** | **WFI** | **StaClear** | **BD** |
|---|---|---|---|
| 2 µm | 4 | 1,980 | 13,200 |
| 5 µm | 2 | 220 | 6.030 |
| 10 µm | 1 | 10 | 1,940 |
| 25 µm | 0 | 0 | 100 |
| 50 µm | 0 | 0 | 40 |

Table 2 below reports the MFI measurements of cumulative particle concentration (number of particles per mL) of particles ≥ 2µm, particles ≥ 5µm, particles ≥ 10µm, particles ≥ 25µm, particles ≥ 50µm between the following solution sources: WFI stock solution in a clean container, BD U100 insulin syringes filled with WFI, and StaClear general use 0.25ml syringe treated according to the method above and filled with WFI.

**Table 2: MicroFlow Imaging**

| **Size** | **WFI** | **StaClear** | **BD** |
|---|---|---|---|
| 2 µm | 16 | 9,300 | 157,380 |
| 5 µm | 5 | 230 | 26,980 |
| 10 µm | 2 | 0 | 3,427 |
| 25 µm | 0 | 0 | 237 |
| 50 µm | 0 | 0 | 3 |

### EXAMPLE 3 - Particle count with Avastin

Testing was performed on 0.25mL polypropylene syringes with an attached 31G needle. Avastin (bevacizumab) was supplied packaged in 4ml glass vials. The syringe types tested were:
- Becton Dickenson U100 insulin syringes,
- Exel U100 insulin syringes, and
- (TL) General use 0.25ml syringes

The general use 0.25ml syringes were processed according to the teachings outlined in Example 1:

32 syringes of each type of syringe were filled with 25 mg/ml of bevacizumab (Avastin^{®}). Particles in solution were measured by micro-flow imaging using a MFI 5200 device. The MFI 5200 device is capable of measuring particles in the size range of 1µm to 70µm, and differentiating the subvisible particles by sub-populations (protein aggregate, silicone micro-droplet, or air bubble).
**a.** Avastin solution from the vial was first tested for particles to get a baseline measurement of particles before filling the solution into syringes. 0.1 ml of Avastin was pipetted from the vial into another clean container. The container was allowed to stand for 1 hour to eliminate any air bubbles. The solution in the clean container was then vortexed at minimum setting using a vortex mixer to suspend any particles back into solution. The solution was then introduced into the MFI instrument for particle measurements. This result gave the baseline particle content in the stock Avastin solution.
**b.** Syringe Testing - Syringes were filled upto the 0.1ml mark with Avastin through the syringe needle.
**c.** Within one minute the Avastin from the filled syringes was expelled into clean and particle free containers.
**d.** After expelling the Avastin solution into containers, they were allowed to stand for 1 hour to eliminate any air bubbles.
**e.** Immediately prior to measurements the fluid was mixed using the a vortex mixer on minimum setting to suspend any resulting particles back into the solution.
**f.** The solution was then introduced into the MFI instrument for particles measurement.

Particle analysis included analyzing images to identify types of particles. Silicone oil micro-droplets have an aspect ratio of ≥ 0.85; and other particles have an aspect ratio <85. The MFI instrument can apply the aspect ratio filters to determine spherical particles which are typically lubricant oil particles or other shapes that would be related to protein aggregates. This aspect ratio filters were used to distinguish between lubricant particles labeled as Silicone and protein aggregates labeled as Other in the figures (Fig 2-7)

FIG. 2 is a bar graph comparing particle concentration (number of particles per mL) of particles ≥ 5µm between the following biologic solution sources: Avastin^{®} straight from the vial, BD U100 insulin syringes filled with Avastin^{®}, Exel U100 insulin syringes with Avastin^{®}, and general use 0.25ml syringe processed according to the teachings of this embodiment and filled with Avastin^{®} (TL). As shown in FIG. 2, the TL general use 0.25ml syringe surprisingly had a total of 1330 particles (silicone particles and other particles) per mL for any particles of a diameter ≥ 5µm.

FIG. 3A a bar graph comparing particle concentration (number of particles per mL) of particles ≥ 10µm between the following biologic solution sources: Avastin^{®} straight from the vial, BD U100 insulin syringes filled with Avastin^{®}, Exel U100 insulin syringes with Avastin^{®}, and TL general use 0.25ml syringe processed according to the method described in this embodiment and filled with Avastin^{®} (TL). As shown in FIG. 3A and FIG 3B (which is an enlarged view of the bar graph in FIG. 3A), the TL general use 0.25ml syringe surprisingly had a total of ≤50 particles (silicone particles and other particles) per mL for any particles of a diameter ≥ 10µm.

FIG. 4A a bar graph comparing particle concentration (number of particles per mL) of particles ≥ 25µm between the following biologic solution sources: Avastin^{®} stock solution from the vial, BD U100 insulin syringes filled with Avastin^{®}, Exel U100 insulin syringes filled with Avastin^{®}, and TL general use 0.25ml syringe processed according to the teachings of this embodiment and filled with Avastin^{®} (TL). As shown in FIG. 4A and FIG 4B (which is an enlarged view of the bar graph in FIG. 4A), the TL general use 0.25ml syringe surprisingly had a total of <7 particles (silicone particles and other particles) per mL for any particles of a diameter ≥ 25µm. Since the Avastin stock solution itself does not meet the USP789 guidelines of number of particles less than 50/ml for sizes ≥ 10 microns and number of particles less than 5/ml for sizes ≥ 25 microns, the Avastin solution would need to be filtered.

### EXAMPLE 4 - Particle Count with Filtered Avastin^{®}

As shown in FIG. 4A, the Avastin^{®} solution in the vial had certain particle counts. In this example, the Avastin^{®} solution from the vial was filtered through a 5µm filter needle prior to filling the syringe.

In this example the 0.25ml general use syringes (TL) processed in accordance to the coating conditions described in Example 1. A total of 64 syringes were divided into two groups. One group was filled with unfiltered Avastin solution from the vial and the second group was filled with the filtered Avastin solution.

Baseline particle level in the Avastin solution before and after filtration were measured. For unfiltered Avastin, 0.1ml was pipetted out of the vial and transferred into a clean particle free container. For filtered Avastin, 0.1ml solution was drawn out of the vial through a BD 5 micron filter needle and then transferred into a clean particle free container. Both containers were allowed to stand for 1 hour to eliminate air bubbles. They were vortexed using a minimum setting to suspend any particles back into solution before introducing them to the MFI for measurement. This constituted the baseline particles for unfiltered and filtered Avastin.

32 TL 0.25ml syringes processed according to the teachings were filled with 25 mg/ml of bevacizumab (Avastin^{®}) up to the 0.1ml mark and 32 of the 0.25ml syringes processed according to the teachings were filled upto the 0.1 ml mark with 25 mg/ml of bevacizumab (Avastin^{®}) filtered through a 5µm filter needle. MFI measurements were performed on these two sets of syringes in accordance to the steps outlined in Example 2.

FIG. 5 a bar graph comparing particle concentration (number of particles per mL) of particles ≥ 5µm between the following biologic solution sources: Avastin^{®} stock solution from the vial, filtered Avastin^{®} solution, general use 0.25ml syringe processed according to the teachings of Example 1 and filled with Avastin^{®} (TL), and general use 0.25ml syringes processed according to the teachings of Example 1 and filled with filtered Avastin^{®} (TL-F). As shown in FIG. 5, the filtered Avastin^{®} had a reduced total particle count as compared to unfiltered Avastin^{®}. Also, general use 0.25ml syringe treated according to the method above and filled with filtered Avastin^{®} surprisingly had a total of 812 particles (silicone particles and other particles) per mL for any particles of a diameter ≥ 5µm.

FIG. 6 a bar graph comparing particle concentration (number of particles per mL) of particles ≥ 10µm between the following biologic solution sources: Avastin^{®} stock solution from the vial, filtered Avastin^{®}from the vial, general use 0.25ml syringe processed according to the teachings of Example 1 and filled with Avastin^{®}, and general use 0.25ml syringe processed according to the teachings of Example 1 and filled with filtered Avastin^{®}. As shown in FIG. 6, the filtered Avastin^{®} had a reduced total particle count as compared to unfiltered Avastin^{®}. Also, general use 0.25ml syringe processed according to the teachings of Example 1 and filled with filtered Avastin^{®} surprisingly had a total of <30 particles (silicone particles and other particles) per mL for any particles of a diameter ≥ 10µm.

FIG. 7 a bar graph comparing particle concentration (number of particles per mL) of particles ≥ 25µm between the following biologic solution sources: Avastin^{®} stock solution from the vial, filtered Avastin^{®} from the vial, general use 0.25ml syringe processed according to the teachings of Example 1 and filled with Avastin^{®} (TL), and general use 0.25ml syringe processed according to the teachings of Example 1 and filled with filtered Avastin^{®} (TL-F). As shown in FIG. 7. the filtered Avastin^{®} had a reduced total particle count as compared to unfiltered Avastin^{®}. Also, general use 0.25ml syringes processed according to the teachings of Example 1 and filled with filtered Avastin^{®} surprisingly had a total of <3 particles (silicone particles and other particles) per mL for any particles of a diameter ≥ 25µm.

### CLAUSES

1. A plastic syringe barrel comprising:
   a plastic syringe barrel treated with plasma and coated with a polysiloxane-based lubricant coating treated with plasma, wherein each plasma treatment consisted essentially of uncharged energized gaseous species; and
   wherein the interior surface of the plasma-treated plastic syringe barrel with the plasma-treated polysiloxane-based lubricant coating has at least a 75% reduction in the number of particles greater than 8 microns in diameter as compared to an interior surface of a plastic syringe barrel with a polysiloxane-based lubricant coating.
2. The plastic syringe barrel of clause 1. wherein the interior surface of the plasma-treated plastic syringe barrel with the plasma-treated polysiloxane-based lubricant coating has at least a 80% reduction in the number of particles greater than 8 microns in diameter as compaied to an interior surface of a plasma-treated plastic syringe barrel with a polysiloxane-based lubricant coating.
3. The plastic syringe barrel of clause 1. wherein the interior surface of the plasma-treated plastic syringe banel with the plasma-neated polysiloxane-based lubricant coating has at least a 95% reduction in the number of particles greater than 8 microns in diameter as compared to an interior surface of a plastic syringe barrel with a plasma-treated polysiloxane-based lubricant coating.
4. A plastic syringe barrel comprising:
   a plastic syringe barrel treated with plasma and coated with a polysiloxane-based lubricant coating treated with plasma, wherein each plasma treatment consisted essentially of uncharged energized gaseous species; and
   wherein the interior surface of the plasma-treated plastic syringe barrel with the plasma-treated polysiloxane-based lubricant coating has a surface density of ≤ 600 particles per 12 cm², wherein the particles are greater than 8 microns in diameter.
5. A plastic syringe barrel comprising:
   a plastic syringe barrel treated with plasma and coated with a polysiloxane-based lubricant coating treated with plasma, wherein each plasma treatment consisted essentially of uncharged energized gaseous species; and
   wherein the interior surface of the plasma-treated plastic syringe barrel with the plasma-treated polysiloxane-based lubricant coating has a surface density of ≤ 50 particles per cm², wherein the particles are greater than 8 microns in diameter.
6. The plastic syringe barrel of clause 5, wherein the interior surface of the plasma-treated plastic syringe barrel with the plasma-treated polysiloxane-based lubricant coating has a surface density of ≤ 40 particles per cm², wherein the particles are greater than 8 microns in diameter.
7. The plastic syringe barrel of clause 5, wherein the interior surface of the plasma-treated plastic syringe barrel with the plasma-treated polysiloxane-based lubricant coating has a surface density of ≤ 35 particles per cm², wherein the particles are greater than 8 microns in diameter.
8. The plastic syringe barrel of clause 5, wherein the interior surface of the plasma-treated plastic syringe barrel with the plasma-treated polysiloxane-based lubricant coating has a surface density of ≤ 30 particles per cm², wherein the particles are greater than 8 microns in diameter.
9. A syringe comprising:
   the plastic syringe barrel of any one of clauses 1-8,
   a plunger rod,
   a plunger stopper, and
   a needle.
10. A syringe comprising:
   the plastic syringe barrel of any one of clauses 1-8,
   a luer lock tip or slip tip,
   a plunger rod, and
   a plunger stopper.
11. The plastic syringe barrel of any one of clauses 1-8, wherein the plastic syringe barrel comprises about 0.005mg/cm² to about 0.5mg/cm² of silicone oil.
12. The plastic syringe barrel of any one of clauses 1-8, wherein the polysiloxane-based lubricant coating is a silicone oil coating.
13. The plastic syringe barrel of any one of clauses 1-8, wherein the polysiloxane-based lubricant coating is polydimethylsiloxane.
14. The syringe of any one of clauses 1-13, wherein the plastic syringe barrel contains a solution.
15. The syringe of any one of clauses 1-13, wherein the plastic syringe barrel contains a solution comprising an anticoagulant, vaccine, or recombinant protein.
16. The syringe of any one of clauses 1-13, wherein is the plastic syringe barrel contains an anti-VEGF protein solution comprising pegaptanib, ranibizumab, aflibercept, or bevacizumab.
17. The syringe of any one of clauses 1-13, wherein the plastic syringe barrel contains an ophthalmic solution.
18. The syringe of clause 17, wherein the particle level in the ophthalmic solution is ≤ 50 particles per ml for any particles ≥ 10µm in diameter or ≤ 5 particles per ml for any particles ≥ 25µm in diameter.
19. The syringe of clause 14, wherein the particle level in the solution is ≤ 50 particles per ml for any particles ≥ 10µm in diameter or ≤ 5 particles per ml for any particles ≥ 25µm in diameter.
20. The plastic syringe barrel of any one of clauses 1-8, wherein the plastic syringe barrel has a maximum fill volume of 1.0ml, 0.5ml, 0.3ml, 0.25ml, 0.10ml, or 0.05ml.
21. A method of treating the eye, comprising intravitreally administering the solution or the ophthalmic solution to an eye with the syringe of any one of clauses 14 -17.
22. A method of producing a plastic syringe barrel with a stable silicone oil layer comprising:
   providing a plastic syringe barrel,
   exposing an interior surface of the plastic syringe barrel to a plasma consisting essentially of uncharged energized gaseous species for 0.1 - 10 seconds;
   applying 0.005 - 0.5mg/cm² of silicone oil to the plasma-treated interior surface of the plastic syringe barrel to form a uniform silicone oil coating; and
   exposing the uniform silicone oil coating to a plasma consisting essentially of uncharged energized gaseous species for 0.1 - 10 seconds;
   wherein the interior surface of the plasma-treated plastic syringe barrel with plasma-treated silicone oil coating has a surface density of ≤ 600 particles per 12 cm², wherein the particles are greater than 8 microns in diameter.
23. A method of producing a plastic syringe barrel with a stable silicone oil layer comprising:
   providing a plastic syringe barrel,
   exposing an interior surface of the plastic syringe barrel to a plasma consisting essentially of uncharged energized gaseous species for 0.1 - 10 seconds;
   applying 0.005 - 0.5mg/cm² of silicone oil to the plasma-treated interior surface of the plastic syringe barrel to form a uniform silicone oil coating; and
   exposing the uniform silicone oil coating to a plasma consisting essentially of uncharged energized gaseous species for 0.1 - 10 seconds;
   wherein the interior surface of the plasma-treated plastic syringe barrel with plasma-treated silicone oil coating has a surface density of ≤ 50 particles per cm², wherein the particles are greater than 8 microns in diameter.
24. The method of clause 23, wherein the interior surface of the plasma-treated plastic syringe barrel with the plasma-treated silicone oil coating has a surface density of ≤ 40 particles per cm², wherein the particles are greater than 8 microns in diameter.
25. The method of clause 23, wherein the interior surface of the plasma-treated plastic syringe barrel with the plasma-treated silicone oil coating has a surface density of ≤ 35 particles per cm², wherein the particles are greater than 8 microns in diameter.
26. The method of clause 23, wherein the interior surface of the plasma-treated plastic syringe barrel with the plasma-treated silicone oil coating has a surface density of ≤ 30 particles per cm², wherein the particles are greater than 8 microns in diameter.
27. A method of producing a plastic syringe barrel with a stable silicone oil layer comprising:
   providing a plastic syringe barrel,
   exposing an interior surface of the plastic syringe barrel to a plasma consisting essentially of uncharged energized gaseous species for 0.1- 10 seconds;;
   applying 0.005 - 0.5mg/cm² of silicone oil to the plasma-treated interior surface of the plastic syringe barrel to form a uniform silicone oil coating; and
   exposing the uniform silicone oil coating to a plasma consisting essentially of uncharged energized gaseous species for 0.1 - 10sec;
   wherein the interior surface of the plasma-treated plastic syringe barrel with the plasma-treated silicone oil coating has at least a 75% reduction in the number of particles greater than 8 microns in diameter as compared to an interior surface of a plastic syringe barrel with a silicone oil coating.
28. The method of clause 27, wherein the interior surface of the plasma-treated plastic syringe barrel with the plasma-treated silicone oil coating has at least a 80% reduction in the number of particles greater than 8 microns in diameter as compared to an interior surface of a plasma-treated plastic syringe barrel with a silicone oil coating.
29. The method of clause 27, wherein the interior surface of the plasma-treated plastic syringe barrel with the plasma-treated silicone oil coating has at least a 95% reduction in the number of particles greater than 8 microns in diameter as compared to an interior surface of a plastic syringe barrel with a plasma-treated silicone oil coating.
30. The method of any one of clauses 22 -29, wherein the uncharged energized gaseous species comprises excited argon gas atoms.
31. The method of any one of clauses 22-29, wherein the plastic is a cyclic olefin polymer (COP), cyclic olefin copolymer (COC), polyethylene (PE), polycarbonate (PC), polypropylene (PP), or polyethylene terephthalate (PET).
32. The method of any one of clause 22-29, wherein the plastic syringe barrel has a maximum fill volume of 1.0ml, 0.5ml, 0.3ml, 0.25ml, 0.10ml, or 0.05ml.
33. The method of any one of clauses 22-29, wherein the plastic is a cyclic olefin polymer (COP)..
34. The method of clause 22, 23 or 27, wherein the method further comprises waiting at least 10 minutes, 20 minutes, 30 minutes, 40 minutes, 1 hour, 2 hours, or 3 hours before exposing the uniform silicone oil coating to the plasma.
35. A method of producing a syringe with a plastic syringe barrel with a stable polysiloxane-based lubricant coating comprising:
   the method of any one of clauses 22-34, and
   assembling the plastic syringe barrel with a plunger rod, plunger stopper, and needle.
36. The method of clause 35, wherein the plastic syringe barrel contains a solution and wherein the particle level in the solution is ≤ 50 particles per ml for any particles ≥ 10µm in diameter or ≤ 5 particles per ml for any particles ≥ 25µm in diameter.
37. A plastic syringe comprising a plastic syringe barrel, a plunger rod, a plunger stopper, wherein the plastic syringe barrel has a stable silicone oil coating and is produced by:
   providing a plastic syringe barrel,
   exposing an interior surface of the plastic syringe barrel to a plasma consisting essentially of uncharged energized gaseous species for 0.1- 10 seconds,;
   applying 0.005 - 0.5 mg/cm² of silicone oil to the plasma-treated interior surface of the plastic syringe barrel to form a uniform silicone oil coating; and
   exposing the uniform silicone oil coating to a plasma consisting essentially of uncharged energized gaseous species;
   wherein the interior surface of the plasma-treated plastic syringe barrel with the plasma-treated silicone oil coating has a surface density of ≤ 600 particles per 12 cm², wherein the particles are greater than 8 microns in diameter.
38. A plastic syringe comprising a plastic syringe barrel, a plunger rod, a plunger stopper, wherein the plastic syringe barrel has a stable silicone oil coating and is produced by:
   providing a plastic syringe barrel,
   exposing an interior surface of the plastic syringe barrel to a plasma consisting essentially of uncharged energized gaseous species for 0.1- 10 seconds,;
   applying 0.005 - 0.5 mg/cm² of silicone oil to the plasma-treated interior surface of the plastic syringe barrel to form a uniform silicone oil coating; and
   exposing the uniform silicone oil coating to a plasma consisting essentially of uncharged energized gaseous species;
   wherein the interior surface of the plasma-treated plastic syringe barrel with the plasma-treated silicone oil coating has a surface density of ≤ 50 particles per cm², wherein the particles are greater than 8 microns in diameter.
39. The plastic syringe of clause 38, wherein the interior surface of the plasma-treated plastic syringe barrel with the plasma-treated polysiloxane-based lubricant coating has a surface density of ≤ 40 particles per cm², wherein the particles are greater than 8 microns in diameter.
40. The plastic syringe of clause 38, wherein the interior surface of the plasma-treated plastic syringe barrel with the plasma-treated polysiloxane-based lubricant coating has a surface density of ≤ 35 particles per cm², wherein the particles are greater than 8 microns in diameter.
41. The plastic syringe of clause 38, wherein the interior surface of the plasma-treated plastic syringe barrel with the plasma-treated polysiloxane-based lubricant coating has a surface density of ≤ 30 particles per cm², wherein the particles are greater than 8 microns in diameter.
42. A plastic syringe comprising a plastic syringe barrel, a plunger rod, a plunger stopper, wherein the plastic syringe barrel has a stable silicone oil coating and is produced by:
   providing a plastic syringe barrel,
   exposing an interior surface of the plastic syringe barrel to a plasma consisting essentially of uncharged energized gaseous species for 0.1- 10 seconds;
   applying 0.005 - 0.5mg/cm² of silicone oil to the plasma-treated interior surface of the plastic syringe barrel to form a uniform silicone oil coating; and
   exposing the uniform silicone oil coating to a plasma consisting essentially of uncharged energized gaseous species;
   wherein the interior surface of the plasma-treated plastic syringe barrel with the plasma-treated silicone oil coating has at least a 75% reduction in the number of particles greater than 8 microns in diameter as compared to an interior surface of a plastic syringe barrel with a silicone oil coating.
43. The plastic syringe of clause 42, wherein the interior surface of the plasma-treated plastic syringe barrel with the plasma-treated silicone oil coating has at least a 80% reduction in the number of particles greater than 8 microns in diameter as compared to an interior surface of a plasma-treated plastic syringe barrel with a silicone oil coating.
44. The plastic syringe of clause 42, wherein the interior surface of the plasma-treated plastic syringe barrel with the plasma-treated silicone oil coating has at least a 95% reduction in the number of particles greater than 8 microns in diameter as compared to an interior surface of a plastic syringe barrel with a plasma-treated silicone oil coating.
45. The plastic syringe of clause 37, 38 or 42, wherein the plastic syringe barrel contains a solution.
46. The plastic syringe of clause 37, 38 or 42, wherein the plastic syringe barrel contains a solution comprising an anticoagulant, vaccine, or recombinant protein.
47. The plastic syringe of clause 37, 38 or 42, wherein the plastic syringe barrel contains an anti-VEGF protein solution comprising pegaptanib, ranibizumab, aflibercept, or bevacizumab.
48. The plastic syringe of clause 37, 38 or 42, wherein the plastic syringe barrel contains an ophthalmic solution.
49. The plastic syringe of clause 45, wherein the particle level in the ophthalmic solution is ≤ 50 particles per ml for any particles ≥ 10µm in diameter or ≤ 5 particles per ml for any particles ≥ 25µm in diameter.
50. The plastic syringe of clause45, wherein the particle level in the solution is ≤ 50 particles per ml for any particles ≥ 10µm in diameter or ≤ 5 particles per ml for any particles ≥ 25µm in diameter.
51. A method of treating the eye, comprising intravitreally administering the solution or the ophthalmic solution to an eye with the plastic syringe of any one of clauses 45-48.
52. A syringe comprising
   a plastic syringe barrel treated with plasma and coated with a polysiloxane-based lubricant coating treated with plasma, wherein each plasma treatment consisted essentially of uncharged energized gaseous species,
   a plunger rod,
   a plunger stopper, and
   a needle;
   wherein the plastic syringe barrel contains a solution and the particle level in the solution is ≤ 50 particles per ml for any particles ≥ 10µm in diameter or ≤ 5 particles per ml for any particles ≥ 25µm in diameter.
53. A method of treating the eye, comprising intravitreally administering the solution to an eye with the syringe of clause 52.
54. The method of clause 53, wherein the solution comprises an anticoagulant, vaccine, or recombinant protein.
55. The method of clause 53, wherein the solution is an ophthalmic solution.
56. The method of clause 53, wherein the solution comprises pegaptanib, ranibizumab, aflibercept, or bevacizumab.
57. A syringe comprising
   a plastic syringe barrel treated with plasma and coated with a polysiloxane-based lubricant coating treated with plasma, wherein each plasma treatment consisted essentially of uncharged energized gaseous species, a luer lock tip or slip tip,
      a plunger rod, and
      a plunger stopper;
      wherein the plastic syringe barrel contains a solution and the particle level in the solution is ≤
   50 particles per ml for any particles ≥ 10µm in diameter or ≤ 5 particles per ml for any particles ≥ 25µm in diameter.
58. A plastic syringe barrel comprising:
   a plastic syringe barrel treated with plasma and coated with a perfluoropolyether lubricant coating treated with plasma, wherein each plasma treatment consisted essentially of uncharged energized gaseous species; and
   wherein the interior surface of the plasma-treated plastic syringe barrel with the plasma-treated perfluoropolyether lubricant coating has at least a 75%, 80%, or 95% reduction in the number of particles greater than 8 microns in diameter as compared to an interior surface of a plastic syringe barrel with a perfluoropolyether lubricant coating.
59. A plastic syringe barrel comprising:
   a plastic syringe barrel treated with plasma and coated with a perfluoropolyether lubricant coating treated with plasma, wherein each plasma treatment consisted essentially of uncharged energized gaseous species; and
   wherein the interior surface of the plasma-treated plastic syringe barrel with the plasma-treated perfluoropolyether lubricant coating has a surface density of ≤ 600 particles per 12 cm², ≤ 500 particles per 12 cm², or ≤ 400 particles per 12 cm², wherein the particles are greater than 8 microns in diameter.
60. A plastic syringe barrel comprising:
   a plastic syringe barrel treated with plasma and coated with a perfluoropolyether lubricant coating treated with plasma, wherein each plasma treatment consisted essentially of uncharged energized gaseous species; and
   wherein the interior surface of the plasma-treated plastic syringe barrel with the plasma-treated perfluoropolyether lubricant coating has a surface density of ≤ 100 particles per cm², ≤ 90 particles per cm², ≤ 50 particles per cm², ≤ 40 particles per cm², ≤ 35 particles per cm², or ≤ 30 particles per cm², wherein the particles are greater than 8 microns in diameter.
61. A syringe comprising:
   the plastic syringe barrel of any one of clauses 58-60,
   a plunger rod,
   a plunger stopper, and
   a needle.
62. A syringe comprising:
   the plastic syringe barrel of any one of clauses 58-60,
   a luer lock tip or slip tip,
   a plunger rod, and
   a plunger stopper.
63. The syringe of any one of clauses 61-62, wherein the plastic syringe barrel contains a solution.
64. The syringe of any one of clauses 61-62, wherein the plastic syringe barrel contains a solution comprising an anticoagulant, vaccine, or recombinant protein.
65. The syringe of any one of clauses 61-62, wherein is the plastic syringe barrel contains an anti-VEGF protein solution comprising pegaptanib, ranibizumab, aflibercept, or bevacizumab.
66. The syringe of any one of clauses 61-62, wherein the plastic syringe barrel contains an ophthalmic solution.
67. The syringe of clause 66, wherein the particle level in the ophthalmic solution is ≤ 50 particles per ml for any particles ≥ 10µm in diameter or ≤ 5 particles per ml for any particles ≥ 25µm in diameter.
68. A method of treating the eye, comprising intravitreally administering the solution or the ophthalmic solution to an eye with the syringe of any one of clauses 61-67.
69. A method of producing a plastic syringe barrel with a stable lubricant layer comprising:
   providing a plastic syringe barrel,
   exposing an interior surface of the plastic syringe barrel to a plasma consisting essentially of uncharged energized gaseous species for 0.1 - 10 seconds;
   applying 0.005 - 0.5mg/cm² of perfluoropolyether to the plasma-treated interior surface of the plastic syringe barrel to form a uniform perfluoropolyether coating; and
   exposing the uniform perfluoropolyether coating to a plasma consisting essentially of uncharged energized gaseous species for 0.1 - 10 seconds;
   wherein the interior surface of the plasma-treated plastic syringe barrel with plasma-treated perfluoropolyether coating has a surface density of ≤ 600 particles per 12 cm², ≤ 500 particles per 12 cm², ≤ 400 particles per 12 cm², ≤ 100 particles per cm², ≤ 90 particles per cm², ≤ 50 particles per cm², ≤ 40 particles per cm², ≤ 35 particles per cm², or ≤ 30 particles per cm²,
   wherein the particles are greater than 8 microns in diameter.
70. A syringe comprising
   a plastic syringe barrel treated with plasma and coated with a perfluoropolyether lubricant coating treated with plasma, wherein each plasma treatment consisted essentially of uncharged energized gaseous species,
   a plunger rod,
   a plunger stopper, and
   a needle;
   wherein the plastic syringe barrel contains a solution and the particle level in the solution is ≤ 50 particles per ml for any particles ≥ 10µm in diameter or ≤ 5 particles per ml for any particles ≥ 25µm in diameter.
71. A method of treating the eye, comprising intravitreally administering the solution to an eye with the syringe of clause 70.
72. The method of clause 71, wherein the solution comprises an anticoagulant, vaccine, or recombinant protein, wherein the solution is an ophthalmic solution, wherein the solution is an anti-VEGF protein solution comprising pegaptanib, ranibizumab, aflibercept, or bevacizumab
73. A syringe comprising a plastic syringe barrel treated with plasma and coated with a perfluoropolyether lubricant coating treated with plasma, wherein each plasma treatment consisted essentially of uncharged energized gaseous species, a luer lock tip or slip tip,
   a plunger rod, and
   a plunger stopper;
   wherein the plastic syringe barrel contains a solution and the particle level in the solution is ≤ 50 particles per ml for any particles ≥ 10µm in diameter or ≤ 5 particles per ml for any particles ≥ 25µm in diameter.

## Claims

1. A syringe (20) comprising
a plastic syringe barrel (1) treated with a downstream plasma and coated with a polysiloxane-based lubricant coating treated with a downstream plasma,
a plunger rod (3),
a plunger stopper (4), and
a needle (6);
wherein the interior surface of the downstream plasma-treated plastic syringe barrel with the downstream plasma-treated polysiloxane-based lubricant coating has a surface density of ≤ 50 particles per cm², wherein the particles are greater than 8 microns in diameter, wherein the plastic syringe barrels are empty when particles are counted, and the syringe contains a solution and the particle level in the solution is ≤ 50 particles per mL for any particles ≥ 10 µm in diameter or ≤ 5 particles per mL for any particles ≥ 25 µm in diameter.

2. The syringe (20) of claim 1, for use in treating the eye.

3. The syringe (20) of claim 2, wherein the solution comprises an anticoagulant, vaccine, or recombinant protein.

4. The syringe (20) of claim 2, wherein the solution is an ophthalmic solution.

5. The syringe (20) of claim 2, wherein the solution comprises an anti-VEGF protein solution comprising pegaptanib, ranibizumab, aflibercept, or bevacizumab.

6. A syringe (20) comprising
a plastic syringe barrel (1) treated with a downstream plasma and coated with a polysiloxane-based lubricant coating treated with a downstream plasma, a luer lock tip (8) or slip tip (9),
a plunger rod (3), and
a plunger stopper (4);
wherein the interior surface of the downstream plasma-treated plastic syringe barrel with the downstream plasma-treated polysiloxane-based lubricant coating has a surface density of ≤ 50 particles per cm², wherein the particles are greater than 8 microns in diameter, wherein the plastic syringe barrels are empty when particles are counted, and the syringe contains a solution and the particle level in the solution is ≤ 50 particles per mL for any particles ≥ 10 µm in diameter or ≤ 5 particles per mL for any particles ≥ 25 µm in diameter.

7. The syringe (20) of claim 6, for use in treating the eye.

8. The syringe (20) of claim 7, wherein the solution comprises an anticoagulant, vaccine, or recombinant protein.

9. The syringe (20) of claim 7, wherein the solution is an ophthalmic solution.

10. The syringe (20) of claim 7, wherein the solution comprises an anti-VEGF protein solution comprising pegaptanib, ranibizumab, aflibercept, or bevacizumab.

11. A method of producing a plastic syringe barrel with a stable silicone oil layer comprising:
providing a plastic syringe barrel (1),
exposing an interior surface of the plastic syringe barrel to a first plasma for 0.1-10 seconds;
applying 0.005-0.5 mg/cm² of silicone oil to the plasma-treated interior surface of the plastic syringe barrel to form a uniform silicone oil coating; and
exposing the uniform silicone oil coating to a second plasma for 0.1-10 seconds;
wherein the interior surface of the first plasma-treated plastic syringe barrel with second plasma-treated silicone oil coating has a surface density of ≤ 50 particles per cm², wherein the particles are greater than 8 microns in diameter and wherein the plastic syringe barrels are empty when particles are counted.

12. The method of claim 11, wherein the interior surface of the plasma-treated plastic syringe barrel with the plasma-treated silicone oil coating has a surface density of ≤ 40 particles per cm², wherein the particles are greater than 8 microns in diameter and wherein the plastic syringe barrels are empty when particles are counted.

13. The method of claim 11, wherein the interior surface of the plasma-treated plastic syringe barrel with the plasma-treated silicone oil coating has a surface density of ≤ 35 particles per cm², wherein the particles are greater than 8 microns in diameter and wherein the plastic syringe barrels are empty when particles are counted.

14. The method of claim 11, wherein the interior surface of the plasma-treated plastic syringe barrel with the plasma-treated silicone oil coating has a surface density of ≤ 30 particles per cm², wherein the particles are greater than 8 microns in diameter and wherein the plastic syringe barrels are empty when particles are counted.

15. A syringe (20) comprising:
the plastic syringe barrel (1) produced by the method of any one of claims 11-14,
a plunger rod (3),
a plunger stopper (4),
a needle (6); and
an ophthalmic solution, wherein the particle level in the ophthalmic solution is ≤ 50 particles per mL for any particles ≥ 10 µm in diameter or ≤ 5 particles per mL for any particles ≥ 25 µm in diameter.
